(19) Europäisches Patentamt
European Patent Office
Office européen des brevets

(11) EP 2 696 963 B1

(12) **EUROPÄISCHE PATENTSCHRIFT**

(45) Veröffentlichungstag und Bekanntmachung des Hinweises auf die Patenterteilung:
**24.03.2021 Patentblatt 2021/12**

(21) Anmeldenummer: **12713932.7**

(22) Anmeldetag: **29.03.2012**

(51) Int Cl.:
***B01D 69/06*** *(2006.01)* ***B01D 71/68*** *(2006.01)*
***B01D 69/02*** *(2006.01)* ***B01D 67/00*** *(2006.01)*
***A61M 5/168*** *(2006.01)* ***A61M 5/14*** *(2006.01)*
***A61M 5/165*** *(2006.01)*

(86) Internationale Anmeldenummer:
**PCT/EP2012/055573**

(87) Internationale Veröffentlichungsnummer:
**WO 2012/139896 (18.10.2012 Gazette 2012/42)**

(54) **MAKROPORÖSE FILTRATIONSMEMBRAN**

MACROPOROUS FILTRATION MEMBRANE

MEMBRANE DE FILTRATION MACROPOREUSE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **13.04.2011 EP 11162177**

(43) Veröffentlichungstag der Anmeldung:
**19.02.2014 Patentblatt 2014/08**

(73) Patentinhaber: **3M Innovative Properties Company**
**St. Paul, MN 55133-3427 (US)**

(72) Erfinder:
- **SCHNEIDER, Frank**
**42349 Wuppertal (DE)**
- **CZERNIK, Martin**
**51379 Leverkusen (DE)**
- **STRIPP, Walter**
**42389 Wuppertal (DE)**
- **TATSCH, Ramona**
**42555 Velbert (DE)**

(74) Vertreter: **Vossius & Partner**
**Patentanwälte Rechtsanwälte mbB**
**P.O. Box 86 07 67**
**81634 München (DE)**

(56) Entgegenhaltungen:
**EP-A1- 2 113 298** **WO-A1-96/40421**
**US-A- 4 943 374** **US-A1- 2004 043 224**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 696 963 B1

## Beschreibung

Die Erfindung betrifft eine hydrophile großporige Filtrationsmembran in Gestalt einer Flachmembran auf Basis eines filmbildenden hydrophoben ersten Polymers aus der Gruppe der aromatischen Sulfonpolymere sowie eines hydrophilen zweiten Polymers, wobei die Membran eine erste und eine zweite Oberfläche aufweist und eine sich zwischen den Oberflächen erstreckende Stützschicht mit einer dreidimensionalen schwammartigen Netzwerkstruktur.

Mikroporöse Polymermembranen werden in verschiedensten Bereichen industrieller, pharmazeutischer oder medizinischer Anwendungen zur Präzisionsfiltration eingesetzt. In diesen Anwendungen gewinnen Membrantrennprozesse zunehmend an Bedeutung, da diese Prozesse den Vorteil bieten, dass die zu trennenden Stoffe thermisch nicht belastet oder gar geschädigt werden. Mikrofiltrationsmembranen ermöglichen beispielsweise die Entfernung feiner Teilchen oder Mikroorganismen mit Größen bis in den Submikronbereich und sind daher für die Herstellung von gereinigtem Wasser für die Verwendung in Laboratorien oder für die Halbleiterindustrie geeignet. Zahlreiche weitere Anwendungen von Membrantrennprozessen sind aus der Getränkeindustrie, der Biotechnologie oder der Abwassertechnologie bekannt.

Zumeist werden dabei Membranen mit einer ausgeprägten Asymmetrie verwendet, die eine Trennschicht besitzen und eine daran angrenzende mikroporöse Stützstruktur mit im Vergleich zur Trennschicht gröberen Poren. Die Poren in der Trennschicht bestimmen dabei die eigentliche Trenncharakteristik der Membran, d.h. durch die Größe der Poren in der Trennschicht wird die Größe der Partikel oder Moleküle kontrolliert, die von der Membran zurückgehalten werden. In der Anwendung werden derartige Membranen vielfach so eingesetzt, dass sie von der offenporigeren Seite angeströmt werden und so die mikroporöse Stützschicht als Vorfilter für die Trennschicht fungiert. Dadurch wird die Schmutzbeladungskapazität der Membran erhöht. Ein Fluid, das die Membran so durchströmt, tritt zuerst in die größeren Poren ein und zuletzt in die kleinen Poren der Trennschicht. Hierdurch werden Partikel, die im Fluid enthalten sind, in der grobporigen Stützschicht zurückgehalten, bevor sie die Trennschicht erreichen und diese verstopfen können.

Für zahlreiche Membrananwendungen stellen Sulfonpolymere wie z.B. Polysulfon oder Polyethersulfon ein vielfach verwendetes Membranmaterial dar, nicht zuletzt wegen ihrer hohen chemischen Stabilität, ihrer Temperaturstabilität oder der Sterilisierbarkeit der daraus hergestellten Membranen. Allerdings handelt es sich bei diesen Polymeren um hydrophobe Polymere, was ihren Einsatz für die Filtration wässriger Medien einschränkt. Darüber hinaus ist bekannt, dass Membranen aus hydrophoben Materialien ein starkes, unspezifisches Adsorptionsvermögen besitzen, woraus im Einsatz häufig eine schnelle Belegung der Membranoberfläche mit vorzugsweise höhermolekularen Bestandteilen der zu filtrierenden Flüssigkeit und in der Folge eine Verschlechterung der Permeabilität resultiert.

Die US-A-6 056 903 betrifft mikroporöse Polyethersulfonmembranen mit einer effektiven Porengröße von mindestens 0,01 μm und bevorzugt einer Porengröße im Bereich von ca. 0,1 bis 20 μm. Die Membranen der US-A-6 056 903 sind jedoch hydrophob und weisen eine über der Wand im wesentlichen symmetrische Porenstruktur ohne Haut auf.

In der EP-A-0 696 935, der US-A-5 846 422 und der US-A-5 979 670 werden ebenfalls Membranen aus Polysulfon beschrieben, die eine ausgeprägte Asymmetrie aufweisen. Durch die alleinige Verwendung des Polyethersulfons sind diese Membranen hydrophob. An ihrer enger porigen Oberfläche weisen sie einen mittleren Porendurchmesser von mindestens 1,2 μm auf, wobei ausgehend von dieser Oberfläche die Membranwand einen isotropen Bereich aufweist, in dem die Poren eine im wesentlichen konstante Größe aufweisen. Die Poren der zweiten Oberfläche sind um mindestens den Faktor 2, vorzugsweise um mindestens den Faktor 3 oder sogar 6 größer als die Poren der ersten Oberfläche. Diese Membranen sollen z.B. bei analytischen oder diagnostischen Verfahren eingesetzt werden, bei denen besonderer Wert auf hohe Durchlaufgeschwindigkeiten gelegt wird. Durch die alleinige Verwendung des Polyethersulfons als die Membranstruktur aufbauendem Polymer sind diese Membranen hydrophob und damit nicht spontan mit Wasser benetzbar. Infolge dieser hydrophoben Eigenschaften neigen diese Membranen in der Anwendung zum Verblocken der inneren Membranstruktur infolge einer Adsorption von in der zu filtrierenden Flüssigkeit enthaltenen Substanzen.

Auch die US-A-5 906 742 betrifft integral asymmetrische Membranen auf Basis von Polysulfonen, deren Wand einen an die erste Oberfläche mit mikroporöser Haut angrenzenden isotropen Bereich mit im wesentlichen konstanten Porendurchmessern aufweist, an den sich zur zweiten Oberfläche hin ein asymmetrischer Bereich mit in Richtung auf die zweite Oberfläche größer werdenden Poren anschließt. Die Membranen der US-A-5 906 742 sind durch Beimischung eines hydrophilen Polymers zum Polysulfon hydrophil. Die Poren in der an den isotropen Bereich angrenzenden mikroporösen Haut haben einen mittleren Durchmesser von mehr als ca. 1 μm, die Poren in der zweiten Oberfläche sind um mehr als den Faktor 2, vorzugsweise um mehr als den Faktor 3 bis 6 oder mehr größer. Ohne Berücksichtigung der Oberflächen ist die Asymmetrie noch größer und beträgt mindestens 10:1 oder 20:1 oder sogar bis zu 100:1 oder 200:1. In ihrer Wand sind also die Membranen der US-A-5 906 742 in hohem Maße asymmetrisch. Die ausgeprägte Asymmetrie in Verbindung mit großen Porengrößen wirkt sich jedoch negativ auf die mechanischen Eigenschaften der Membranen aus und führt insbesondere zu geringen Kompressibilitäten dieser Membranen.

Die US-A-5 886 059 betrifft eine Polyethersulfonmembran für die Mikrofiltration, wobei die Membran eine ausgeprägt asymmetrische Struktur besitzt. Sie weist eine Haut mit relativ kleinen Poren an ihrer einen, ersten Seite auf und, ausgehend von dieser Seite, über die gesamte Membranwand hinweg zunehmende Porengrößen bis hin zur

anderen zweiten Membranseite, wobei die Poren auf der zweiten Seite um den Faktor 50 bis 10000 größer sind als die Poren in der Haut auf der ersten Seite. Membranen mit einer derartigen Struktur sind zum einen anfällig gegen mechanische Beschädigungen bezüglich der Schicht mit den kleinsten Poren, d.h. der Trennschicht, die sich an der Oberfläche der Membran befindet. Zum anderen weisen die Membranen aufgrund der spezifischen asymmetrischen Struktur nur eine mäßige mechanische Stabilität auf.

Die WO 02/07854 offenbart ein Blutsammelsystem mit einem Hauptfilter zur Entfernung von Leukozyten. Der Hauptfilter besteht aus einer Membran aus Polyethersulfon, die einen schichtförmigen Aufbau und eine erste Hautoberfläche und eine zweite Hautoberfläche sowie eine innere Porenstruktur mit zellförmigen Poren aufweist, wobei die Membran ebenfalls eine ausgeprägt asymmetrische Porenstruktur über der Wand aufweist. Die zur ersten Hautoberfläche benachbarten Zellen der inneren Porenstruktur sind kleiner als die zur zweiten Hautoberfläche benachbarten Zellen der inneren Porenstruktur, wobei die Mehrzahl der Poren in der ersten Oberfläche einen Durchmesser zwischen etwa 12 und 28 $\mu$m aufweist.

Anwendungen im Bereich der Medizintechnik betreffen unter anderem auch Geräte für diagnostische Zwecke, wie sie z.B. in der US-A-5 240 862 beschrieben werden. Bei den Geräten der US-A-5 240 862 handelt es sich um Systeme zur Blutauftrennung, bei denen eine asymmetrische Membran im innigen Kontakt mit einer Sammelmembran ist. In der Anwendung wird Vollblut auf die grobporigere Seite der asymmetrischen Membran aufgebracht, und Blutzellen werden in den großen Poren der asymmetrischen Membran zurückgehalten. Blutplasma tritt durch die Membran hindurch und tritt in die Sammelmembran ein. Die Sammelmembran ist in der Regel mit einem analytischen Nachweissystem ausgerüstet, so dass das Plasma auf bestimmte darin enthaltene Bestandteile hin untersucht werden kann.

Eine spezifische Anwendung von mikroporösen Membranen in der Medizintechnik betrifft Filter für die Applikation, d.h. die Verabreichung von Infusionslösungen. Hier werden offenporige Membranfilter in den Strömungsweg der Infusionslösung eingebaut, um so die Durchflussrate von Infusionslösungen, wie z.B. von wässrigen Natriumchlorid-, Glukose-, und Lipidlösungen, unabhängig von der hydrostatischen Druckhöhe konstant zu halten und auch eventuell in der Infusionslösung enthaltene Partikel oder ungelöste Stoffe zurückzuhalten. Gleichzeitig haben diese Membranfilter oftmals die Aufgabe, Gasblasen aus Infusionslösungen abzutrennen. Derartige Filter werden beispielsweise in der EP 0 254 100 B1 offenbart.

In der US 4,943,374 B1 wird ein Verfahren zur Abtrennung von schädlichen Keimen, Trübstoffen und Hefe aus Bier offenbart, umfassend das Filtern von Bier, das diese Stoffe enthält, mittels einer mikroporösen Membran, die aus Polyethersulfon und einem Hydrophilisierungsmittel aufgebaut ist, und die eine Porengröße im Bereich von 0.2 bis 0.8 Mikrometern aufweist.

Die WO 96/40421 A1 beschreibt eine Sulfonpolmyermembrane, die durch simultanes Gießen mit einem hydrophilen Polymer erhalten wurde, und die eine mikroporöse Oberfläche und eine poröse Stützschicht umfasst, wobei die mikroporöse Oberfläche eine hohe Porendichte und die poröse Stützschicht eine isotropische Region von substantiell konstanter Porengröße und eine asymmetrische Region von graduell zunehmender Porengröße aufweist.

In der EP 2 113 298 A1 wird eine semipermeable Hohlfasermembran offenbart, wobei das Membranmaterial eine Polyethersulfon oder ein Polysulfon und ein Polyvinylpyrrolidone umfasst, wobei das Polyvinylpyrrolidon aus einem Komponente mit einem niedrigen Molekulargewicht von weniger als 100 kDa und einer Komponente mit einem hohen Molekulargewicht von 100 KDa oder mehr besteht, und wobei die Membran einen Siebkoeffizienten für Myoglobin in wässriger Lösung zwischen 86 % und 90 % aufweist.

Die US 2010/0219122 A1 offenbart eine zur Behandlung von Flüssigkeiten geeignete Hohlfasermembran, wobei das Verhältnis von Flux 120 zu Flux 30 0.45 oder höher ist, wenn die Filtration durch zuführen einer Lösung mit einer Trübung von 20 NTU in eine Hohlfasermembran mit einer internen Durchmesser von 500 bis 1500 Mikrometern und einer Membrandicke von 100 bis 500 Mikrometern mit einem Transmembrandruck von 1.5 bar durchgeführt wird, und der Flux 30 nach 30 Minuten nach dem Beginn der Filtration flux ist, und der Flux 120 nach 120 Minuten nach dem Beginn der Filtration flux ist.

In der JP 2008-221050 A1 wird eine semipermeable Hohlfasermembran beschrieben, umfassend eine poröse Schicht aus einem Polyarylatharz und einem Polyethersulfonharz, eine innere dichte Schicht und eine äußere dichte Schicht. Dabei weisen die innere dichte Oberflächenschicht und die äußere dichte Oberflächenschicht jeweils geringere Luftwirkungsgrade als die poröse Schicht auf. Die erste dichte Schicht und die zweite dichte Schicht weisen einen mittleren Porendurchmesser von 2 bis 300 nm auf.

Bei der Verabreichung von Infusionslösungen besteht jedoch vielfach der Wunsch, dass am Ende der Applikation eine Flüssigkeitssäule in der Infusionskanüle stehen bleibt und eine neue Infusionsflaschen angeschlossen werden kann, ohne dass eine Entlüftung der Kanüle erforderlich wäre. Hierdurch ließe sich das Problem der Entlüftung von Infusionskanülen und/oder der zu verabreichenden Infusionslösungen zumindest verringern. Es besteht daher ein Bedarf an einem Filterelement, welches neben Filtrationseigenschaften in Bezug auf eventuelle Verunreinigungen in Infusionslösungen die Eigenschaft besitzt, ein Leerlaufen von Infusionskanülen zu verhindern. Durch das Filterelement soll bei Einbau des Filterelements in den Strömungsweg der wässrigen Flüssigkeit bzw. der Infusionslösung ein Abreißen der ausgebildeten Flüssigkeitssäule vermieden und die Ausbildung genügend hoher Flüssigkeitssäulen beispielsweise in

Kapillaren ermöglicht werden, die unterhalb eines solchen Filterelements angeordnet sind, so dass also eine genügende Flüssigkeitssäule im Infusionsschlauch stehen bleibt. Gleichzeitig soll das Filterelement einen geringen hydraulischen Widerstand bei der Durchströmung mit Flüssigkeiten aufweisen. Darüber hinaus soll es eine hohe mechanische Stabilität aufweisen. Es ist daher Aufgabe der vorliegenden Erfindung, ein für derartige Anwendungen geeignetes Filterelement zur Verfügung zu stellen.

Die Aufgabe wird durch eine hydrophile großporige Filtrationsmembran in Gestalt einer Flachmembran auf Basis eines filmbildenden hydrophoben ersten Polymers aus der Gruppe der aromatischen Sulfonpolymere sowie eines hydrophilen zweiten Polymers gelöst, wobei die Membran aus einer ersten und einer zweiten Oberfläche und einer sich zwischen den Oberflächen erstreckende Stützschicht mit einer dreidimensionalen schwammartigen Netzwerkstruktur besteht,

- wobei die Stützschicht an ihrer der ersten Oberfläche zugewandten Seite eine erste Deckschicht und an ihrer der zweiten Oberfläche zugewandten Seite eine zweite Deckschicht aufweist, die integral mit der Stützschicht ausgebildet sind, und
- wobei die erste und die zweite Oberfläche näherungsweise ovale oder kreisförmige Öffnungen aufweisen, die die erste bzw. zweite Deckschicht durchdringen und mit der Stützschicht in Verbindung stehen, wobei der mittlere Durchmesser der Öffnungen in der zweiten Oberfläche 0,5 bis 1,5 mal so groß wie der mittlere Durchmesser der Öffnungen in der ersten Oberfläche ist und unter der Bedingung, dass der mittlere Durchmesser der Öffnungen in der ersten Oberfläche und der mittlere Durchmesser der Öffnungen in der zweiten Oberfläche sich um weniger als den Faktor 2 unterscheiden,
- wobei die dreidimensionale Netzwerkstruktur der Stützschicht aus dicken Verästelungen und einem Porensystem mit durchgehenden Poren aufgebaut ist und mindestens 70 % der Verästelungen an ihrer dünnsten Stelle einen Durchmesser durch Bestimmung anhand von Rasterelektronenmikroskop-Aufnahmen von Querschnitten der Membranen in einer Vergrößerung von mindestens 1000 von mindestens 0,5 μm haben, wobei die dreidimensionale Netzwerkstruktur keine kavernenartigen Poren, die auch als Fingerporen bezeichnet werden, enthält;
- wobei die Poren in der Stützschicht größer sind als die Öffnungen in den Oberflächen;
- wobei die Filtrationsmembran eine Dicke im Bereich zwischen 50 und 150 μm aufweist; und

der mittlere Durchmesser der Öffnungen in der zweiten Oberfläche im Bereich von 2 bis 20 μm liegt.

Die erfindungsgemäßen Membran weist auf Grund der sie aufbauenden Polymere, d.h. dadurch, dass ihre Struktur aus einem Polymer aus der Gruppe der aromatischen Sulfonpolymere sowie aus einem hydrophilen zweiten Polymer aufgebaut ist, hydrophile Eigenschaften auf und ist mit wässrigen Flüssigkeiten spontan benetzbar, d.h. ein auf die Membranoberfläche aufgetropfter Wassertropfen wird sofort von der Membran aufgesogen. Damit sind die Membranen für den Einsatz bei z.B. der Filtrierung von wässrigen Flüssigkeiten hervorragend geeignet.

Die vorliegende Membran basiert auf einem hydrophoben ersten Polymer aus der Gruppe der aromatischen Sulfonpolymere und enthält darüber hinaus ein hydrophiles zweites Polymer. Als aromatische Sulfonpolymere kommen im Rahmen der vorliegenden Erfindung z. B. Polysulfone, Polyethersulfone, Polyphenylensulfone, Polyarylethersulfone oder Copolymere oder Modifikationen dieser Polymere oder Mischungen dieser Polymere untereinander in Frage.

In einer bevorzugten Ausführungsform ist das hydrophobe erste Polymer ein Polysulfon oder ein Polyethersulfon mit den in nachstehenden Formeln (I) und (II) dargestellten wiederkehrenden Moleküleinheiten. Besonders bevorzugt wird als hydrophobes erstes Polymer ein Polyethersulfon gemäß Formel (II) eingesetzt, da dieses eine geringere Hydrophobie aufweist als beispielsweise das Polysulfon.

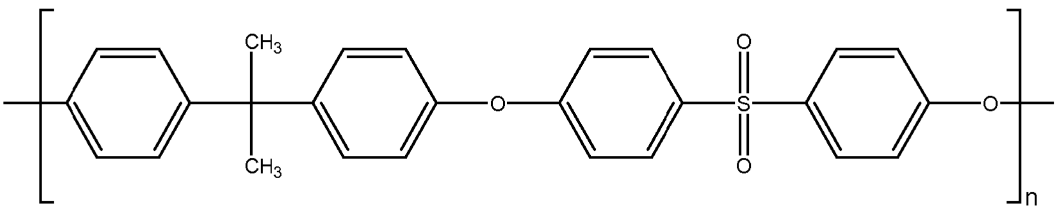

( I )

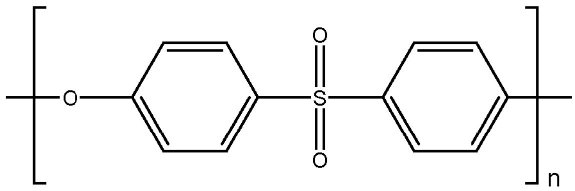

( II )

Als hydrophiles zweites Polymer werden vorteilhafterweise langkettige Polymere eingesetzt, die eine gute Kompatibilität zu dem hydrophoben ersten Polymer aufweisen und die über wiederkehrende polymere Einheiten verfügen, die an sich hydrophil sind. Bevorzugt sind solche hydrophilen Polymere, die ein mittleres Molekulargewicht Mw von mehr als 10 000 Dalton aufweisen. Die als hydrophile zweite Polymere eingesetzten Polymere haben gleichzeitig im Verfahren zur Herstellung der erfindungsgemäßen Membranen die Aufgabe, die Viskosität der homogenen Spinnlösung zu erhöhen, d.h. als Verdicker zu fungieren, weshalb diese Polymere vielfach auch als Verdicker bezeichnet werden. Daneben wirken diese Polymere bei der Ausbildung der Membranstruktur auch als Porenbildner oder Nukleierungsmittel. Vorzugsweise ist das hydrophile zweite Polymer ein Polyvinylpyrrolidon, ein Polyethylenglykol, ein Polyvinylalkohol, ein Polyglykolmonoester, ein Polysorbitat, wie z.B. Polyoxyethylensorbitanmonooleat, ein Polyacrylat, eine Carboxylmethylcellulose, eine Polyacrylsäure oder eine Modifikation oder ein Copolymer dieser Polymere. Besonders bevorzugt ist das hydrophile zweite Polymer ein Polyvinylpyrrolidon. Es ist in einer weiteren bevorzugten Ausführungsform auch möglich, Mischungen von verschiedenen hydrophilen Polymeren einzusetzen und insbesondere Mischungen von hydrophilen Polymeren mit unterschiedlichen Molekulargewichten, z.B. Mischungen von Polymeren, deren Molekulargewicht sich um den Faktor 5 oder mehr unterscheidet. Vorzugsweise beträgt die Konzentration des hydrophilen zweiten Polymers in der erfindungsgemäßen Membran 0,5 bis 7 Gew.-%, bezogen auf das Gewicht der Membran. Diese Polymere können ggf. in der Membran noch chemisch oder physikalisch modifiziert werden. So kann Polyvinylpyrrolidon nachträglich z.B. durch Bestrahlung mit energiereicher Strahlung vernetzt und damit wasserunlöslich gemacht werden.

Zur Modifikation der Oberflächeneigenschaften der Membranen können Additive eingesetzt werden, welche die Stabilität der Membran, die Farbe, das Adsorptions- oder Absorptionsvermögen beeinflussen. Es sind auch Zusätze möglich, die die Ladung der Membran regeln, z.B. der Membran anionischen oder kationischen Charakter verleihen. Vorzugsweise kann die Membran des Weiteren ein hydrophiles drittes Polymer enthalten, welches von dem hydrophilen zweiten Polymer verschieden und besonders bevorzugt ein hydrophil modifiziertes aromatisches Sulfonpolymer ist. Durch das Vorhandensein eines solchen Polymers könnnen insbesondere die Permeabilität der Membran sowie deren Adsorptionseigenschaften günstig beeinflusst werden, und die Membranen könnnen eine permanente Hydrophilie aufweisen, die sich u.a. darin äußert, dass die Membran mehrfach dampfsterilisierbar ist und auch nach beispielsweise 30 Sterilisationszyklen die hydrophilen Eigenschaften i.w. unverändert erhalten bleiben. In einer besonders bevorzugten Ausführungsform kann das hydrophil modifizierte aromatische Sulfonpolymer in der Membran in einer Konzentration von 1 bis 50 Gew.-%, bezogen auf das Gewicht der Membran, vorliegen, wobei die Summe der Polymere sich zu 100 Gew.-% ergibt. Hierzu umfasst im Verfahren zur Herstellung der bevorzugten erfindungsgemäßen Membranen die Polymerkomponente des Weiteren ein hydrophiles drittes Polymer, welches von dem hydrophilen zweiten Polymer verschieden und ein hydrophil modifiziertes aromatisches Sulfonpolymer ist.

Bei dem hydrophil modifizierten aromatischen Sulfonpolymer kann es sich um ein solches handeln, bei dem hydrophile funktionelle Gruppen kovalent an das Sulfonpolymer gebunden sind. Es kann sich aber auch um ein Copolymer auf Basis eines Sulfonpolymers handeln, in dem hydrophile Segmente enthalten sind, beispielsweise um ein Copolymer aus einem Sulfonpolymer mit einem hydrophilen Polymer wie z.B. Polyvinylpyrrolidon oder Polyethylenglykol. Aus Gründen der Kompatibilität ist es von besonderem Vorteil, wenn das hydrophil modifizierte aromatische Sulfonpolymer auf dem hydrophoben ersten aromatischen Sulfonpolymer basiert, d.h. die Membranstruktur enthält eine Mischung aus einem hydrophoben ersten aromatischen Sulfonpolymer und einer hydrophilen Modifikation dieses Polymers. Sehr gute Ergebnisse werden erzielt, wenn es sich bei dem hydrophil modifizierten aromatischen Sulfonpolymer um ein sulfoniertes Sulfonpolymer handelt, wobei dieses sulfonierte Sulfonpolymer vorzugsweise einen Sulfonierungsgrad im Bereich von 3 bis 10 % aufweist. Membranen, die eine Kombination aus Polyethersulfon und sulfoniertem Polyethersulfon enthalten, weisen besonders hohe Permeabilitäten für Wasser und Proteine sowie eine geringe Neigung zur Adsorption z.B. von Proteinen o.ä. und damit ein geringes Fouling auf.

Die Stützschicht der vorliegenden Membranen weist eine dreidimensionale Netzwerkstruktur auf, die aus dicken Verästelungen und einem durchgehenden Porensystem mit relativ großen Poren aufgebaut ist und einer dreidimensionalen Korallenstruktur ähnelt, wobei die Membranen jedoch keine kavernenartigen Poren, die auch als Fingerporen bezeichnet werden, enthält. Dabei haben mindestens 70 % der Verästelungen an deren dünnster Stelle einen Durchmesser im Bereich von mindestens 0,5 μm und vorzugsweise von mindestens 2 μm. Bei einer genaueren Untersuchung der Verästelungen mittels Rasterelektronenmikroskopie zeigt sich, dass diese, wie dies auch für die Deckschichten gilt, an ihrer Außenseite eine Haut und in ihrem Inneren eine poröse Struktur besitzen, wobei die Haut sowohl der Deckschichten als auch der Verästelungen in der Stützstruktur bei Untersuchung mittels Rasterelektronenmikroskopie glatt, gleichmäßig und ebenmäßig ist und bei 500facher Vergrößerung keine Unebenheiten wie z.B. Grate, Krater und ähnliches erkennbar sind. Auf Grund dieser Struktur der Stützschicht weisen die Membranen einen geringen hydraulischen Widerstand auf, da die Stützstruktur aufgrund der relativ großen Poren trotz des großen Anteils der Stützschicht an der Gesamtdicke der Membrane höchstens einen geringen Betrag zum hydraulischen Widerstand der Membran bei der Durchströmung mit wässrigen Medien leistet.

In einer bevorzugten Ausführungsform weist in diesem Zusammenhang die Stützschicht Poren mit einem Durchmesser von mindestens 1/10 der Membrandicke auf. Die Dicke der vorliegenden Membranen liegt im Bereich zwischen 50 und 150 µm. Es zeigt sich, dass bei derartigen Dicken der vorliegenden Membran das generelle Niveau des Durchströmwiderstandes auf ausreichend niedrigem Niveau liegt und gleichzeitig die mechanische Stabilität der Membran auch im Hinblick auf ihre Handhabung während ihrer Verarbeitung zur Herstellung von Filtern ausreichend hoch ist. Es wurde überraschender Weise auch gefunden, dass Membranen mit höheren Membrandicken zunehmend eine über die Membranwand ausgeprägtere asymmetrische Struktur mit deutlichen Unterschieden hinsichtlich der mittleren Durchmesser der Öffnungen in den beiden Membranoberflächen aufweisen, die sich um mehr als den Faktor 2 unterscheiden. Darüber hinaus ist festzustellen, dass sich bei höheren Membranwanddicken die Permeabilitäten der Membranen deutlich abnehmen. Durch den Aufbau der Membran, d.h. durch ihre Struktur bzw. ihrer Morphologie sowie durch ihre geometrischen Abmessungen werden ein niedriges Niveau des Durchströmwiderstandes und damit hohe Permeabilitäten für die vorliegende Membran erzielt. Hohe Permeabilitäten sind jedoch erforderlich, damit z.B. bei der Verabreichung von Infusionslösungen wegen der relativ geringen Druckhöhen oberhalb der Membran genügend hohe Durchflussraten erreicht werden. Vorzugsweise weist die vorliegende Membran einen die Permeabilität kennzeichnenden Transmembranfluss für Wasser, $TMF_{H2O}$, im Bereich von 700 bis 4000 ml/($cm^2$·min·bar) und besonders bevorzugt im Bereich von 1500 bis 4000 ml/($cm^2$·min·bar) auf.

Es hat sich gezeigt, dass die Verästelungen, die die Netzwerkstruktur ausbilden und die zum überwiegenden Anteil an ihrer dünnsten Stelle einen Durchmesser im Bereich von mindestens 0,5 µm haben, gleichzeitig für eine hohe mechanische Stabilität der Membran z.B. gegenüber Druckbeanspruchungen oder auch gegenüber Zugbelastungen sorgen. Unter dem überwiegenden Anteil wird im Rahmen der vorliegenden Erfindung verstanden, dass mindestens 70% der Verästelungen an ihrer dünnsten Stelle einen Durchmesser von mindestens 0,5 µm aufweisen. Als Grundlage für die Beurteilung können dabei Rasterelektronenmikroskop (REM)-Aufnahmen von Querschnitten der Membranen in einer Vergrößerung von mindestens 1000, vorzugsweise von mindestens 1500 dienen, anhand derer die Verästelungen ausgemessen werden können.

Die für die erfindungsgemäße Membran typische Struktur mit den die Netzwerkstruktur ausbildenden relativ dicken Verästelungen ist - wie ausgeführt wurde - unmittelbar über Rasterelektronenmikroskopaufnahmen oder auch über Transmissions-Elektronenmikroskopaufnahmen von Membranquerschnitten feststellbar und lässt sich hierüber mit Strukturen von anderen Membranen vergleichen. Membranen mit dicken Verästelungen werden beispielsweise in der EP-A-0 882 494 B1 beschrieben, wobei es sich bei den dortigen Membranen um asymmetrische Hohlfasermembranen mit von der Außenseite zu Innenseite allmählich ansteigenden Porendurchmessern handelt. Auch die EP-A-0 297 744 offenbart mikroporöse Strukturen mit einer dreidimensionalen Netzwerkstruktur, deren Verästelungen einen Durchmesser bis in den Bereich von 1 µm aufweisen. Die Durchmesser und Längen der Verästelungen werden anhand von Rasterelektronenmikroskopaufnahmen ermittelt oder alternativ über bildanalytische Auswerteverfahren. Des Weiteren befasst sich auch die EP-A-0 543 355 B1 mit Membranen mit netzwerkartigen Strukturen, wobei die in dieser Schrift offenbarten Membranen Netzwerkstrukturen mit vergleichsweise feinen Verästelungen oder Fibrillen aufweisen, die Durchmesser im Bereich von 0,1 bis 0,5 µm besitzen.

In einer bevorzugten Ausführungsform haben die vorliegenden Membranen eine auf ihren Querschnitt bezogene Zugfestigkeit von mindestens 500 cN/$mm^2$ und besonders bevorzugt von 700 cN/$mm^2$ auf. Ebenso sind die vorliegenden Membranen trotz der relativ groben Netzwerkstruktur bzw. relativ dicken Verästelungen nicht spröde, sondern besitzen eine vergleichsweise hohe Bruchdehnung, wobei vermutet wird, dass auch die innere Porenstruktur in den Verästelungen bzw. den Deckschichten dafür ursächlich ist. Bevorzugt beträgt die Bruchdehnung in mindestens einer Erstreckungsrichtung der Membran mindestens 30 % und besonders bevorzugt mindestens 40 %. Durch die besondere dreidimensionale Netzwerkstruktur mit dicken Verästelungen lässt sich eine hohe mechanische Stabilität auch bei relativ hohen Volumenporositäten der Membranen erreichen. Bevorzugt liegt die Volumenporosität im Bereich von 65 bis 85 Vol.-%.

Die Membran weist an ihrer ersten Oberfläche und an ihrer der zweiten Oberfläche jeweils eine Deckschicht auf, die integral mit der Stützschicht ausgebildet ist. Über die Deckschichten der erfindungsgemäßen Membran lässt sich der Druckverlust bzw. der hydraulische Widerstand der Membran bei der Durchströmung mit wässrigen Flüssigkeiten beeinflussen. Gleichzeitig hat sich gezeigt, dass über die Ausbildung der Deckschichten und insbesondere über die Größe der in den Deckschichten vorhandenen Öffnungen bzw. Poren die Höhe von Flüssigkeitssäulen beeinflusst werden kann, die in Kapillaren unterhalb von Filterelementen verbleiben, die von den vorliegenden Membranen gebildet werden, nachdem eine durch die Filter hindurchgeleitete Flüssigkeit vollständig durch diesen hindurchgeströmt ist, die Durchströmung also zum Stillstand gekommen ist. Es zeigt sich, dass mittels der vorliegenden Membranen in Infusionsschläuchen unterhalb der Membranelemente Wassersäulen einer Höhe von mindestens 1 m realisiert werden können, ohne dass es zu einem Durchbruch von Luft durch die Membran und damit zu einem Abreißen der Flüssigkeitssäule unterhalb der Membran kommt.

Die erste bzw. die zweite Deckschicht besitzt vorzugsweise eine Dicke im Bereich von ca. 3 bis ca. 10 μm. Bei Deckschichten mit einer Dicke von weniger als ca. 3 μm wird eine hinreichende Stabilität der Deckschichten nicht mehr erreicht und eine durchgängige Ausbildung der Deckschichten ist nicht mehr gewährleistet. Bei Dicken von mehr als ca. 10 μm kann der Druckverlust bei der Durchströmung der Deckschichten zu hoch werden und die hydraulische Permeabilität der Membran ist dann zu gering. Die Dicke der Deckschichten kann z.B. anhand von REM-Aufnahmen von Querschnitten der erfindungsgemäßen Membranen durch Ausmessen ermittelt werden, wobei REM-Aufnahmen mit einer Vergrößerung von 250 oder 500 geeignet sind.

Es wird vermutet, dass auch die Oberflächenporen einen Einfluss auf Kapillarkräfte in der Membran haben, wenn diese mit wässrigen Flüssigkeiten benetzt ist. Dabei hat sich gezeigt, dass es von Vorteil ist, wenn die Öffnungen bzw. Poren in den Oberflächen der vorliegenden Membran einen gleichförmigen, im Mittel näherungsweise ovalen oder kreisförmigen Querschnitt aufweisen. Unter näherungsweise kreisförmigen Öffnungen bzw. Poren werden dabei im Rahmen der vorliegenden Erfindung Öffnungen verstanden, bei denen das Verhältnis der aufeinander senkrecht stehenden und durch den Öffnungs- bzw. Porenmittelpunkt gehenden größten Achse und kleinsten Achse der Öffnung bzw. Poren nicht größer als 2 ist. Gleichzeitig ist es von Vorteil, dass die Öffnungen bzw. Poren in den Oberflächen der vorliegenden Membranen eine Insel-im-Meer-Struktur (island-sea-structure) aufweisen, die gleichförmigen, im Mittel näherungsweise ovalen oder kreisförmigen Öffnungen in den Oberflächen also Inseln in der Deckschicht ausbilden und jeweils von Deckschichtmaterial umgeben sind. Die Deckschichten bilden jeweils eine kontinuierliche Phase in Form von die Öffnungen bzw. Poren umschließenden Stegen aus, die an der jeweiligen Oberfläche in einer Ebene liegen. Unmittelbar unterhalb der Oberflächen mit näherungsweise ovalen oder kreisförmigen Öffnungen geht die Membranstruktur in die dreidimensionale Nezwerkstruktur mit unregelmäßig ausgebildetem Porensystem über.

Damit unterscheidet sich die vorliegende Membran hinsichtlich ihrer Oberflächenstruktur von Membranen, bei denen die netzwerkartige Struktur der Stützschicht bis an mindestens eine der Oberflächen heranreicht, so dass die Oberflächen selbst eine netzwerkartige oder eine fibrillenartige Oberflächenstruktur aufweisen, wie dies beispielsweise in der US-A-6 054 899 für die offenporige Seite der dort beschriebenen ausgeprägt asymmetrischen Membranen, für die Innenseite der Hohlfasermembranen der EP-A-361 085 oder für die in der EP-A-0 543 355 offenbarten Membranen gezeigt wird.

Mit Blick auf die Anwendungen der vorliegenden Membran und insbesondere auch zur Erzielung hinreichend hoher Durchflussraten durch die Membran beträgt in einer bevorzugten Ausgestaltung der poröse Flächenanteil der ersten Oberfläche, d.h. der Flächenanteil der Öffnungen bzw. Poren in der ersten Oberfläche an der Gesamtfläche der ersten Oberfläche mindestens 20% und besonders bevorzugt mindestens 30%. Um eine ausreichende mechanische Stabilität der Membran und auch eine hinreichende Kapillarwirkung der Membran zu erreichen, ist es vorteilhaft, wenn der poröse Flächenanteil der ersten Oberfläche kleiner als 70% und besonders vorteilhaft, wenn er kleiner als 60% ist. Der Anteil an Poren in der zweiten Oberfläche ist in der Regel geringer als der poröse Flächenanteil in der ersten Oberfläche und liegt bei mindestens ca. 5%, vorzugsweise bei mindestens 10%.

Wie bereits ausgeführt wurde, besitzt die Membran in der ersten und der zweiten Oberfläche näherungsweise ovale oder kreisförmige Öffnungen, die die erste bzw. zweite Deckschicht durchdringen und mit der Stützschicht in Verbindung stehen. Dabei unterscheidet sich der mittlere Durchmesser der Öffnungen in der ersten Oberfläche vom mittleren Durchmesser der Öffnungen in der zweiten Oberfläche um weniger als den Faktor 2. Gleichzeitig erscheinen die Öffnungen in den Oberflächen kleiner als die Poren in der Stützschicht. Der mittlere Durchmesser der Öffnungen in der zweiten Oberfläche liegt im Bereich von 2 bis 20 μm. Im Unterschied zu mikroporösen Membranen des Stands der Technik ist also die vorliegende Membran in Bezug auf die Porengröße an den Oberflächen im wesentlichen symmetrisch.

Die integral mit der Stützschicht ausgebildeten Deckschichten an der ersten Oberfläche und an der zweiten Oberfläche der Membran weisen Poren auf, die im Mittel kleiner sind als die Poren in der Stützschicht. Die hat zum einen zu Folge, dass die Deckschichten auch einen Beitrag zum Durchflusswiderstand bei der Durchströmung der Membran mit einer Flüssigkeit liefern. Die im Hinblick auf eine bestimmte Trennaufgabe, d.h. auf die Abtrennung von Bestandteilen einer bestimmten Größe aus einer Flüssigkeit, kleinsten Poren der Membran (Trennporen), befinden sich also in den Deckschichten bzw. innerhalb derjenigen Deckschicht, die die kleineren Poren aufweist. Mit Blick auf Anwendungen, bei denen Aufgaben der Membran im Bereich der Filtration liegen, weisen die vorliegenden Membranen in einer bevorzugten Ausführungsform einen mittels der Blaspunktmethode bestimmten Durchmesser der maximalen Trennpore $d_{max}$ im Bereich von 2 bis 20 μm auf und besonders bevorzugt im Bereich von 5 bis 10 μm.

In einer weitern bevorzugten Ausführungsform der vorliegenden Membran ist der Durchmesser der Poren in der Stützschicht, ausgehend von der ersten Deckschicht in Richtung auf die zweite Deckschicht über mindestens 50 % der Dicke der Stützschicht in einem ersten Bereich der Stützschicht im wesentlichen konstant. Der erste Bereich der Stützschicht stellt also einen im Hinblick auf die Porenstruktur im wesentlichen isotropen Bereich dar. Dabei wird im Rahmen der vorliegenden Erfindung unter einem im wesentlichen isotropen Bereich ein Bereich der Membranwand mit

einer im wesentlichen konstanten Porengröße verstanden, wobei eine Beurteilung anhand von Rasterelektronenmikroskop- oder Transmissions-Elektronenmikroskopaufnahmen erfolgt. Der isotrope Bereich kann auch als ein Bereich mit sich durch die Membranwand erstreckenden Strömungskanälen mit im wesentlichen konstantem mittleren Durchmesser angesehen werden. Wie bei jeder Membran variiert auch bei der erfindungsgemäßen Membran die aktuelle Porengröße etwas, d.h. sie weist eine gewisse Porengrößenverteilung auf, wenn auch die Porengrößenverteilung visuell isotrop erscheint. Daher sind von der Erfindung auch Ausführungen des im wesentlichen isotropen Bereichs umfasst, bei denen sich die Porengröße maximal um ca. 15-20% ändert.

In einem der zweiten Deckschicht zugewandten zweiten Bereich der Stützschicht kann in einer bevorzugten Ausführungsform der Durchmesser der Poren durchweg größer sein als im ersten Bereich. In einer weiteren bevorzugten Ausführungsform nimmt der Porendurchmesser in einem sich in Richtung auf die zweite Deckschicht an den ersten Bereich der Stützschicht anschließenden zweiten Bereich zunächst zu, durchläuft ein Maximum und nimmt dann in Richtung auf die zweite Deckschicht auf den in der zweiten Deckschicht vorliegenden Porendurchmesser ab. Dabei ist gleichzeitig der Anteil der Poren in der Struktur in der Stützschicht und insbesondere der zweiten Deckschicht voneinander verschieden, wie sich bereits aus dem porösen Flächenanteil der zweiten Deckschicht im Vergleich zur Gesamtporosität der Membran ergibt.

Die vorliegenden Membranen sind besonders für Anwendungen in der Medizintechnik geeignet, wie z.B. als Filterelement in Tropfkammern von Infusionsbestecken für die Applikation, d.h. die Verabreichung von Infusionslösungen. Bei derartigen Anwendungen ermöglicht die Membran es, dass die Durchflussmenge von Infusionslösungen, wie z.B. von wässrigen Natriumchlorid-, Glukose-, und Lipidlösungen, unabhängig von der dabei auftretenden hydrostatischen Druckhöhe konstant bleibt und dass gleichzeitig am Ende der Applikation eine Flüssigkeitssäule im Infusionsschlauch stehen bleibt. Dabei wirkt sich die die Deckschichten und die Verästelungen der vorliegenden Membran überziehende Haut auf Grund ihrer glatten, ebenmäßigen Struktur positiv in Richtung einer geringen Adsorption von Inhaltsstoffen der Infusionslösungen wie z.B. von Lipiden, Aminosäuren, Kohlenhydraten, Lactaten usw. aus. Die erfindungsgemäßen Membran befindet sich dabei als Filterlement im Bereich des Ausgangs der Tropfkammer in den mit der Tropfkammer verbundenen Infusionsschlauch der Infusionsbestecke.

Die Erfindung betrifft daher auch ein Infusionsbesteck zur Verabreichung von Infusionslösungen, umfassend eine Tropfkammer und einen mit der Tropfkammer verbundenen Infusionsschlauch, bei welchem im Bereich des Ausgangs der Tropfkammer in den Infusionsschlauch die erfindungsgemäße Membran eingebracht ist.

Die vorliegenden Membranen lassen sich durch ein Verfahren mit Nichtlöserinduzierter Phasenseparation herstellen. Ein solches Verfahren zur Herstellung der erfindungsgemäßen Membran umfasst die Schritte:

a. Herstellung einer homogenen Gießlösung aus einer Polymerkomponente, umfassend ein hydrophobes erstes Polymer aus der Gruppe der aromatischen Sulfonpolymere und ein hydrophiles zweites Polymer sowie gegebenenfalls ein hydrophiles drittes Polymer, in einem Lösemittelsystem, wobei die Gießlösung 10 bis 25 Gew.-%, vorzugsweise 12 bis 18 Gew.-%, bezogen auf das Gewicht der Lösung, des hydrophoben ersten Polymers, 2 bis 20 Gew.-%, vorzugsweise 5 bis 18 Gew.-%, bezogen auf das Gewicht der Lösung, des hydrophilen zweiten Polymers sowie bei Vorhandensein des hydrophilen dritten Polymers dieses in einer Konzentration im Bereich von 0,2 bis 20 Gew.-%, vorzugsweise 0,5 bis 15 Gew.-%, bezogen auf das Gewicht der Gießlösung, umfasst und das Lösemittelsystem aus 5 bis 80 Gew.-%, vorzugsweise 10 bis 60 Gew.-%, bezogen auf das Gewicht der Lösung, eines Lösemittels für die Polymerkomponente, 0 bis 80 Gew.-%, vorzugsweise 5 bis 60 Gew.-%, bezogen auf das Gewicht der Lösung, latentem Lösemittel für die Polymerkomponente sowie 0 bis 70 Gew.-%, vorzugsweise 0 bis 40 Gew.-%, bezogen auf das Gewicht der Lösung, Nichtlöser für die Polymerkomponente besteht,
b. Temperieren der homogenen Gießlösung auf eine Ausformtemperatur,
c. Ausgießen der homogenen Gießlösung zu einem Film auf einen temperierbaren Träger,
d. Hindurchführen des auf dem Träger befindlichen Films durch eine klimatisierte Zone,
e. Einführen des auf dem Träger befindlichen Films in eine Koagulationsflüssigkeit und Einleiten der Koagulation des Films zur Ausbildung einer Membranstruktur,
f. Abziehen der Membranstruktur vom Träger mittels einer mit einer Abzugseinrichtung innerhalb der Koagulationsflüssigkeit,
g. Stabilisierung der Membranstruktur im Koagulationsmedium,
h. Extraktion der so erhaltenen Membran und anschließende Trocknung der Membran,

wobei die Gießlösung über Auswahl und Konzentration der in ihr enthaltenen Komponenten so einzustellen ist, dass sie bei einer Temperatur von 40°C eine Viskosität von mindestens 8 Pa s aufweist und die einzelnen

Verfahrensbedingungen so einzustellen sind, dass die erfindungsgemäße Membran mit ihren in Anspruch 1 definierten Eigenschaften erhalten wird.

Als hydrophobes erstes Polymer aus der Gruppe der aromatischen Sulfonpolymere sind die zuvor genannten Polymere einzusetzen, auf denen die erfindungsgemäße Membran basiert. Gleiches gilt für das hydrophile zweite Polymer sowie für das gegebenenfalls eingesetzte hydrophile dritte Polymer. Das zur Herstellung der Gießlösung verwendete Lösungsmittelsystem ist auf das membranbildende Sulfonpolymer abzustimmen. Vorzugsweise umfasst das Lösungsmittelsystem polare aprotische Lösungsmittel, wie z.B. Dimethylformamid, Dimethylacetamid, Dimethylsulfoxid, N-Methylpyrrolidon oder deren Mischungen, oder protische Lösemittel wie etwa ε-Caprolactam. Ferner kann das Lösungsmittelsystem bis zu 80 Gew.-% an latentem Lösemittel enthalten, wobei unter einem latenten Lösemittel im Rahmen der vorliegenden Erfindung ein solches verstanden wird, das das Sulfonpolymer nur schlecht oder erst bei erhöhter Temperatur löst. Im Fall der bevorzugten Verwendung von ε-Caprolactam als Lösemittel können beispielsweise γ-Butyrolacton, Propylencarbonat oder Polyalkylenglykol eingesetzt werden. Darüber hinaus kann das Lösungsmittelsystem Nichtlöser für das membranbildende Polymere enthalten, wie z.B. Wasser, Glycerin, niedermolekulare Polyethylenglykole mit einem Gewichtsmittel des Molekulargewichts von weniger als 1000 Dalton oder niedermolekulare Alkohole, wie etwa Ethanol oder Isopropanol.

Für die Durchführung des Verfahrens zur Herstellung der erfindungsgemäßen Membran ist es wichtig, dass die Viskosität der Gießlösung auf einen Wert oberhalb von 8 Pa s eingestellt wird, und von Vorteil, wenn sie auf einen Wert oberhalb von 10 Pa s eingestellt wird, wobei die Viskosität bei 60°C zu ermitteln ist. Die Einstellung der Viskosität kann insbesondere durch die Konzentration des im erfindungsgemäßen Verfahren eingesetzten hydrophilen zweiten Polymers erfolgen, wobei gleichzeitig auch die Art und das Molekulargewicht des hydrophilen zweiten Polymers von Einfluss sind. Im Übrigen wirkt sich aber auch das eingesetzte Lösemittelsystem auf die Viskosität der Gießlösung aus, also z.B. Art oder Anteil an Lösemittel, latentem Lösemittel und Nichtlöser. Beispielsweise lassen sich bei Verwendung von ε-Caprolactam als Lösemittel vor allem auch in Kombination mit γ-Butyrolacton, Propylencarbonat oder Polyalkylenglykol als latentem Lösemittel Viskositäten im erforderlichen Bereich realisieren.

Es wurde in Untersuchungen festgestellt, dass die Viskosität der Gießlösung zusammen mit der Dicke der herzustellenden Membran entscheidenden Einfluss auf die Struktur der Membran hat, worunter insbesondere die Ausprägung der dreidimensionalen Netzwerkstruktur und der Oberflächen mit den darin befindlichen Poren bzw. Öffnungen zu verstehen sind. Die hohe Viskosität der Gießlösungen in Verbindung mit dem erfindungsgemäß geforderten Bereich bezüglich der Dicke der Membran hat sich als wichtig für die Ausbildung einer dreidimensionalen Netzwerkstruktur mit dicken Verästelungen und einer im wesentlichen symmetrischen Struktur über der Wanddicke erwiesen, bei der sich die mittleren Durchmesser der Öffnungen in den Oberflächen um weniger als den Faktor 2 unterscheiden und bei der im bevorzugten Fall der Durchmesser der Poren, ausgehend von der ersten Deckschicht in Richtung auf die zweite Deckschicht, über mindestens 50 % der Dicke der Stützschicht in einem ersten Bereich der Stützschicht im wesentlichen konstant ist.

Membranen aus Lösungen mit niedrigeren Viskositäten unterhalb von 8 Pa s und mit höheren Membrandicken weisen eine über die Membranwand ausgeprägte asymmetrische Struktur auf mit deutlicheren Unterschieden hinsichtlich der mittleren Durchmesser der Öffnungen in den beiden Membranoberflächen, die sich dann um mehr als den Faktor 2 unterscheiden.

Das Ausgießen der Gießlösung zu einem Film kann auf an sich bekannte Weise erfolgen, beispielsweise mittels üblicher Formwerkzeuge wie Breitschlitzdüsen, Gießkästen oder Rakel. Spätestens im Formwerkzeug wird die Gießlösung auf die Ausformtemperatur eingestellt. Für die Herstellung der erfindungsgemäßen Membranen hat es sich als günstig herausgestellt, wenn die Temperatur der dem Formwerkzeug zugeführten Gießlösung im wesentlichen mit der Temperatur des Formwerkzeugs übereinstimmt und um nicht mehr als ca. 20°C, vorzugsweise um nicht mehr als 15°C von dieser abweicht. Dabei hat es sich als vorteilhaft erwiesen, wenn das Formwerkzeug eine Temperatur von mindestens 50°C aufweist.

Das Ausgießen der Gießlösung erfolgt auf einen temperierbaren Träger, wobei auch hier auf übliche Träger zurückgegriffen werden kann, vom dem die koagulierte Membran im späteren abgezogen werden kann. Beispielsweise können beschichtete Papiere oder Metallbänder eingesetzt werden. Vorzugsweise handelt es sich bei dem temperierbaren Träger um eine temperierbare Heizwalze, d.h. Gießwalze, auf die der Film ausgegossen wird. Die Temperatur des temperierbaren Trägers wird vorzugsweise auf einen Wert eingestellt, der maximal um ca. ± 20 % von der Ausformtemperatur der Gießlösung abweicht. Dies ist von Vorteil, um zum einen die Viskosität der Gießlösung im erforderlichen Bereich zu halten, und zum anderen, um eine ausreichende Offenporigkeit der resultierenden Membran zu gewährleisten.

Zur Erzeugung der nahezu symmetrischen Struktur ist es des Weiteren erforderlich, den auf dem Träger befindlichen Film durch eine klimatisierte Zone zu führen, in der eine definierte Temperatur und eine definierte relative Luftfeuchtigkeit eingestellt ist. Vorzugsweise liegt die Temperatur in der klimatisierten Zone im Bereich zwischen 40 und 60°C, und besonders bevorzugt im Bereich zwischen 45 und 55°C. Die relative Luftfeuchtigkeit wird bevorzugt auf Werte im Bereich von 40 bis 75% eingestellt. Die Verweilzeit des Films in der klimatisierten Zone sowie die Überströmgeschwindigkeit der Luft in der klimatisierten Zone über dem ausgegossenen Film ist so zu bemessen, dass eine Vorkoagulation durch Aufnahme der als Nichtlöser wirkenden Luftfeuchtigkeit an der dem temperierten Träger abgewandten Seite der Gießlösung, d.h. an der Luftseite induziert wird. Die Luftseite wird im späteren die zweite Oberfläche der erfindungsgemäßen Filtrationsmembran. Bevorzugt liegt die Verweilzeit des Films in der klimatisierten Zone oberhalb

von 10 s und besonders bevorzugt oberhalb von 15 s.

Nach Durchlaufen der klimatisierten Zone wird der auf dem Träger befindliche Film in eine Koagulationsflüssigkeit geführt und eine Koagulation zur Ausbildung der Membranstruktur eingeleitet. Hierbei ist zur Erzielung der erfindungsgemäßen Membranstruktur die Koagulationsflüssigkeit auf eine Temperatur zu temperieren, die um 0 bis 60°C niedriger als die Temperatur des temperierbaren Trägers ist. Für die Herstellung von erfindungsgemäßen Membranen mit hohen Transmembranflüssen ist die Koagulationsflüssigkeit bevorzugt auf eine Temperatur temperiert, die um 20 bis 60°C niedriger als die Temperatur des temperierbaren Trägers ist, und besonders bevorzugt auf eine Temperatur, die um 35 bis 60°C niedriger als die Temperatur des temperierbaren Trägers ist.. In einer vorteilhaften Ausgestaltung des erfindungsgemäßen Verfahrens ist die Koagulationsflüssigkeit Wasser bzw. ein Wasserbad. Bevorzugt liegt die Verweilzeit des Films in der Koagulationsflüssigkeit in der gleichen Größenordnung wie in der klimatisierten Zone und damit oberhalb von 10 s und besonders bevorzugt oberhalb von 15 s.

In der Koagulationsflüssigkeit wird der Film zunächst soweit zur Membranstruktur ausgefällt, dass diese bereits eine hinreichende Stabilität aufweist und vom Träger, d.h. vorzugsweise von der Gießwalze abgezogen werden kann. Im Anschluss an die Abzugseinrichtung wird in nachfolgenden Koagulationsbädern die Koagulation vervollständigt und die Membran stabilisiert. Diese Koagulationsbäder können gegenüber dem ersten, zuvor beschriebenen Koagulationsbad eine höhere Temperatur aufweisen. Die Temperatur kann auch von Bad zu Bad in Stufen erhöht werden. Dabei erfolgt in den Koagulationsbädern gleichzeitig eine Extraktion des Lösemittelsystems und in der Regel von Teilen des hydrophilen zweiten Polymers aus der Membranstruktur, so dass die Koagulationsbäder gleichzeitig als Wasch- oder Extraktionsbäder fungieren. Als Koagulations- bzw. Waschmedium in diesen Koagulations- bzw. Waschbädern wird bevorzugt Wasser eingesetzt.

Nach der Extraktion wird die erhaltene Membran beispielsweise mittels eines Walzentrockners getrocknet und die trockene Membran danach aufgewickelt. Während der Extraktion und Trocknung der Membran ist ebenfalls eine geringfügige Verstreckung von Vorteil, um bestimmte Membraneigenschaften wie z.B. die Oberflächenporosität und die Trenneigenschaften definiert einzustellen.

Die Erfindung wird nachfolgend an Hand der nachfolgenden Beispiele und Figuren näher erläutert, wobei der Umfang der Erfindung durch die Beispiele nicht eingeschränkt wird.

Es zeigen:

Fig. 1: Rasterelektronenmikroskopische (REM-) Aufnahme eines Querschnitts der Membran gemäß Beispiel 1 bei 250-facher Vergrößerung.

Fig. 2: REM-Aufnahme eines Ausschnitts des Querschnitts der Membran gemäß Beispiel 1 im Bereich der Membranseite, die bei der Herstellung der Gießwalze zugewandt war (Walzenseite), bei 2000-facher Vergrößerung.

Fig. 3: REM- Aufnahme der Oberfläche der Membran gemäß Beispiel 1, die bei der Herstellung der Gießwalze zugewandt war (Walzenseite) bei 100-facher Vergrößerung.

Fig. 4: REM- Aufnahme der Oberfläche der Membran gemäß Beispiel 1, die bei der Herstellung der Gießwalze abgewandt war (Luftseite) bei 100-facher Vergrößerung.

Fig. 5: REM- Aufnahme der Oberfläche der Membran gemäß Beispiel 1, die bei der Herstellung der Gießwalze zugewandt war (Walzenseite) bei 500-facher Vergrößerung.

Fig. 6: REM- Aufnahme der Oberfläche der Membran gemäß Beispiel 1, die bei der Herstellung der Gießwalze abgewandt war (Luftseite) bei 500-facher Vergrößerung.

Fig. 7: REM-Aufnahme des Gesamtquerschnitts der Membran gemäß Beispiel 1 bei ca. 1600-facher Vergrößerung.

Fig. 8: REM-Aufnahme eines Querschnitts der Membran gemäß Beispiel 2 bei 250-facher Vergrößerung.

Fig. 9: REM-Aufnahme des Querschnitts der Membran gemäß Beispiel 2 im Bereich der Membranseite bei 500-facher Vergrößerung.

Fig. 10: REM- Aufnahme der Oberfläche der Membran gemäß Beispiel 2, die bei der Herstellung der Gießwalze zugewandt war (Walzenseite) bei 100-facher Vergrößerung.

Fig. 11: REM- Aufnahme der Oberfläche der Membran gemäß Beispiel 2, die bei der Herstellung der Gießwalze abgewandt war (Luftseite) bei 100-facher Vergrößerung.

Fig. 12: REM-Aufnahme des Gesamtquerschnitts der Membran gemäß Beispiel 2 bei ca. 1600-facher Vergrößerung.

Fig. 13: REM-Aufnahme eines Querschnitts der Membran gemäß Beispiel 3 bei 250-facher Vergrößerung.

Fig. 14: REM-Aufnahme des Querschnitts der Membran gemäß Beispiel 3 im Bereich der Membranseite bei 500-facher Vergrößerung.

Fig. 15: REM- Aufnahme der Oberfläche der Membran gemäß Beispiel 3, die bei der Herstellung der Gießwalze zugewandt war (Walzenseite) bei 100-facher Vergrößerung.

Fig. 16: REM- Aufnahme der Oberfläche der Membran gemäß Beispiel 3, die bei der Herstellung der Gießwalze abgewandt war (Luftseite) bei 100-facher Vergrößerung.

Fig. 17: REM-Aufnahme eines Querschnitts der Membran gemäß Vergleichsbeispiel 1.

Fig. 18: REM- Aufnahme der Oberfläche der Membran gemäß Vergleichsbeispiel 1, die bei der Herstellung der Gießwalze zugewandt war (Walzenseite).

Fig. 19: REM- Aufnahme der Oberfläche der Membran gemäß Vergleichsbeispiel 1, die bei der Herstellung der Gießwalze abgewandt war (Luftseite).

Fig. 20: REM-Aufnahme eines Querschnitts der Membran gemäß Vergleichsbeispiel 2.

Fig. 21: REM- Aufnahme der Oberfläche der Membran gemäß Vergleichsbeispiel 2, die bei der Herstellung der Gießwalze zugewandt war (Walzenseite).

Fig. 22: REM- Aufnahme der Oberfläche der Membran gemäß Vergleichsbeispiel 2, die bei der Herstellung der Gießwalze abgewandt war (Luftseite).

Fig. 23: REM-Aufnahme eines Querschnitts der Membran gemäß Vergleichsbeispiel 3b bei 100-facher Vergrößerung.

Fig. 24: REM-Aufnahme des Querschnitts der Membran gemäß Vergleichsbeispiel 3b bei 250-facher Vergrößerung.

Fig. 25: REM- Aufnahme der Oberfläche der Membran gemäß Vergleichsbeispiel 3b, die bei der Herstellung der Gießwalze zugewandt war (Walzenseite), bei 500-facher Vergrößerung.

Fig. 26: REM- Aufnahme der Oberfläche der Membran gemäß Vergleichsbeispiel 3b, die bei der Herstellung der Gießwalze abgewandt war (Luftseite), bei 500-facher Vergrößerung.

Fig. 27: REM-Aufnahme eines Querschnitts der Membran gemäß Vergleichsbeispiel 4.

Fig. 28: REM- Aufnahme der Oberfläche der Membran gemäß Vergleichsbeispiel 4, die bei der Herstellung der Gießwalze zugewandt war (Walzenseite).

Fig. 29: REM- Aufnahme der Oberfläche der Membran gemäß Vergleichsbeispiel 4, die bei der Herstellung der Gießwalze abgewandt war (Luftseite).

Fig. 30: REM-Aufnahme eines Querschnitts der Membran gemäß Vergleichsbeispiel 6.

Die folgenden Methoden zur Charakterisierung der Membranen werden angewandt:

Ermittlung der Viskosität der Gießlösung:

Die Viskosität der Gießlösung wird bei einer Temperatur von 60°C mittels eines Rotationsrheometers (Rheo-Stress 1, Fa. Haake) unter Verwendung einer Zylinder-Messeinrichtung Z20 DIN ermittelt. Die Messung erfolgt bei einer Scherrate von 10 $s^{-1}$.

Ermittlung der Volumenporosität:

Es werden vier Proben von ca. 15 $cm^2$ der zu untersuchenden Membran eingewogen und in ca. 50 ml Wasser über 16 h gelagert. Anschließend werden die Proben aus dem Wasser genommen und überschüssiges Wasser mittels Löschpapier entfernt. Die so vorbehandelten Proben werden zur Ermittlung des Nassgewichts gewogen und danach bei 50°C über 16 h getrocknet. Nach Abkühlung wird das Gewicht der getrockneten Proben (Trockengewicht) ermittelt.

Die Volumenporosität wird aus dem Mittelwert der Wasseraufnahme (Nassgewicht minus Trockengewicht), bezogen auf den Mittelwert des Trockengewichts der Proben, unter Verwendung der Dichten für Wasser und das die Membranstruktur aufbauende Polymers (hydrophobes erstes Polymer) ermittelt.

Ermittlung des Transmembranflusses (Wasserpermeabilität):

Aus der zu prüfenden Membran werden scheibenförmige Membranproben ausgestanzt und in einen geeigneten Probenhalter am Umfang fluiddicht so eingespannt, dass eine freie Messfläche von 17,35 $cm^2$ resultiert. Der Probenhalter befindet sich in einem Gehäuse, das von Wasser druckbeaufschlagt durchströmt werden kann. Die eingespannte Membranprobe wird dann von der Seite her, auf der sich die Oberfläche der Membran mit den kleineren Poren befindet, von auf 25°C temperiertem, vollentsalztem Wasser unter einem definierten Druck zwischen 0,1 und 0,2 bar durchströmt. Es wird das während einer Messzeit von 60 s durch die Membranprobe hindurch geströmte Wasservolumen gravimetrisch oder volumetrisch ermittelt.

Der Transmembranfluss TMF wird nach der Formel (III)

$$TMF \left[\frac{l}{m^2 \cdot h \cdot bar}\right] = \frac{V_W}{\Delta t \cdot A_M \cdot \Delta p} \cdot 600 \qquad (III)$$

ermittelt. Hierbei sind:

- $V_W$ = durch die Membranprobe während der Messzeit hindurch geströmte Wasservolumen [ml]
- $\Delta t$ = Messzeit [min]
- $A_M$ = durchströmte Fläche der Membranprobe (17,35 $cm^2$)
- $\Delta p$ = eingestellter Druck während der Messung [bar]

Ermittlung der maximalen Trennpore:

Der Durchmesser der maximalen Trennpore wird mittels der Blaspunktmethode (ASTM Nr. 128-61 und F 316-86) bestimmt, wozu beispielsweise die in der DE-A-36 17 724 beschriebene Methode geeignet ist. Dabei ergibt sich $d_{max}$ aus dem zum Blaspunkt zugehörigen Gasraumdruck $P_B$ nach der Beziehung

$$d_{max} = \sigma_B / P_B$$

Hierbei ist $\sigma_B$ eine Konstante, die hauptsächlich von der bei der Messung eingesetzten benetzenden Flüssigkeit abhängig ist. Für Wasser beträgt $\sigma_B$ bei 25°C 2,07 $\mu m \cdot bar$.

Ermittlung von Bruchkraft bzw. Bruchfestigkeit:

Die Messung der Bruchkraft der Membranen erfolgt mit einer üblichen Universalprüfmaschine der Fa. Zwick, Ulm. Hierzu werden aus der zu untersuchenden Flachmembran Proben herausgeschnitten, deren Kanten in Produktionsrichtung sowie quer zur Produktionsrichtung orientiert sind. Die Proben haben eine Breite von 15 mm und werden so in die Prüfmaschine eingespannt, dass eine freie Länge von 25 cm resultiert.

Die Membranproben werden mit konstanter Geschwindigkeit in Längsrichtung bzw. in Querrichtung der Probe bis zum Bruch gedehnt. Die dafür benötigte Kraft wird in Abhängigkeit der Längenänderung gemessen und in einem Kraft-Dehnungsdiagramm festgehalten. Die Messung erfolgt als Mehrfachbestimmung an fünf Membranproben bei 100 mm Einspannlänge und bei einer Zuggeschwindigkeit von 500 mm/min. Das Vorspanngewicht beträgt 2,5 cN. Die zum Bruch benötigte Kraft BK wird als gemittelter Zahlenwert in cN und die dabei erreichte Bruchdehnung in % der Ausgangslänge ausgegeben.

Die Bruchfestigkeit $\sigma_B$ der Membranprobe in $cN/mm^2$ wird durch Normierung der Bruchkraft BK auf die Querschnittsfläche $A_Q$ der Membranwand erhalten, die sich aus der Probenbreite und der Membrandicke ergibt.

Untersuchung der von der Membran gehaltenen Flüssigkeitssäule:

Aus der zu untersuchenden Flachmembran wird eine Probe mit einem Durchmesser von ca. 1,8 cm ausgestanzt. Die Probe wird an ihrem äußeren Rand so in einen Probenhalter fluiddicht eingebettet, dass im Zentrum eine freie Fläche mit einem Durchmesser von 1,2 cm (1,13 $cm^2$ Messfläche) resultiert. Der Probenhalter wird mit seinem oberen Ende mit einem Einlassstutzen und mit seinem unteren Ende mit einem Auslassstutzen versehen. Mit dem Auslassstutzen wird das obere Ende eines flexiblen Schlauchs mit einem Innendurchmesser von 3 mm und einer Länge von ca. 1,75 m verbunden. Das untere Ende des Schlauchs wird in einer Höhe von 1 m unterhalb der Membranprobe positioniert und mündet offen in einen Auffangbehälter. Der Einlassstutzen wird mit einem Wasserbehälter verbunden, der ca. 1 l fasst.

Mittels einer Rollenklemme wird der flexible Schlauch zunächst abgeklemmt und der Wasserbehälter oberhalb des Probenhalters mit Wasser befüllt. Zu Beginn der Untersuchung wird die Rollenklemme geöffnet und der Wasserdurchfluss durch die Membranprobe sowie den Schlauch gestartet. Nach Durchlauf der gesamten vorgelegten Wassermenge wird geprüft, ob in dem Schlauch unterhalb der Membran eine Wassersäule von 1 m Höhe durch die Membranprobe gehalten wird.

Beispiel 1:

In einem beheizbaren Kessel wurden als Lösemittelsystem 33 kg $\gamma$-Butyrolacton, 33 kg $\varepsilon$-Caprolactam, 4,2 kg Glycerin und 0,8 kg Wasser vorgelegt und zu einer homogenen Flüssigkeit gemischt. In diesem Lösemittelsystem wurden unter Rühren zunächst 1,3 kg sulfoniertes Polyethersulfon (SPES) mit einem Sulfonierungsgrad von 5% innerhalb 1 h gelöst und anschließend 15,22 kg Polyethersulfon (PES, Typ Ultrason E6020, Fa. BASF) unter Rühren eingestreut und während 4 h gelöst. Danach wurden 12,50 kg Polyvinylpyrrolidon (PVP, Typ K30, Fa. ISP) feinverteilt eingerührt und homogenisiert. Durch Anlegen von Vakuum und Beaufschlagung mit Stickstoff wurde der Sauerstoff aus dem Kessel weitgehend entfernt. Hiernach wurde der Kessel auf 80°C aufgeheizt und unter intensivem Rühren während 36 h eine homogene Gießlösung hergestellt. Danach wurde die Gießlösung auf 70°C abgekühlt und mittels Vakuum entgast. Die erhaltene Lösung hatte bei 40°C eine Viskosität von 11,6 Pa s.

Die fertige Gießlösung wurde mittels eines auf 80°C temperierten Gießkastens auf einer auf 65°C temperierte Metall-Gießwalze zu einem Film mit einer Dicke von ca. 130 $\mu$m ausgegossen. Der auf der Gießwalze befindliche Film wurde durch eine Klimazone geführt und während ca. 26 s mit einem Klima von 50°C und 63% rel. Luftfeuchte (Taupunkt ca. 40°C) beaufschlagt, bevor er in ein Koagulationsbad mit auf 65°C temperiertem Wasser eingeführt wurde. Nach einer Verweilzeit von 26 s zur Ausbildung der Membranstruktur wurde der Film mittels einer Abzugswalze von der Gießwalze abgezogen. In nachfolgenden Waschbädern wurde die Membran bei stufenweise auf 90°C ansteigenden Temperaturen in Wasser fixiert und das Lösemittel zusammen mit Großteilen des PVP extrahiert. Die Trocknung der Membran erfolgte mittels eines Walzentrockners.

Die so hergestellte Membran war permanent hydrophil und spontan mit Wasser benetzbar und hatte eine Dicke von ca. 95 $\mu$m sowie eine mittels Blaspunktmethode bestimmte maximale Trennpore von 6,9 $\mu$m. Sie wies einen Transmembranfluss von ca. 1320 ml/($cm^2$·min·bar) sowie eine Porosität von ca. 81 Vol.-% auf. Für die Membran wurde eine mittlere Bruchfestigkeit von 770 $cN/mm^2$ in Längsrichtung (Produktionsrichtung) und von 760 $cN/mm^2$ in Querrichtung gemessen. Die mittlere Bruchdehnung betrug in Längsrichtung 28% und in Querrichtung 47%. Im Test hielt die Membran problemlos eine Wassersäule von mindestens einem Meter.

Fig. 1 zeigt einen Querschnitt der Membran des Beispiels 1, bei dem an den Oberflächen der Membran sowohl an der Walzenseite (rechts in der in Fig. 1 gezeigten REM-Aufnahme) als auch an ihrer Luftseite (links in der in Fig. 1 gezeigten REM-Aufnahme) eine Poren aufweisende Deckschicht zu erkennen ist. Zwischen den Deckschichten erstreckt sich eine grobporige Stützschicht, wobei sich an die der Walzenseite zugeordnete Deckschicht ein erster Bereich der Stützschicht anschließt, in dem die Porengröße im wesentlichen konstant ist und der sich - ausgehend von der Walzenseite - über ca. 70 % der Stützschicht erstreckt. Im einem zweiten Bereich der Stützschicht, der sich bis zu der der Luftseite zugeordneten Deckschicht erstreckt, erscheint die Porenstruktur grobporiger als im ersten Bereich der Stützschicht.

Anhand der Fig. 2, die bei 2000-facher Vergrößerung einen Ausschnitt des Membranquerschnitts im Bereich derjenigen Membranseite zeigt, die bei der Herstellung der Membran der Gießwalze abgewandt war (Luftseite), , ist erkennbar, dass die Membranstruktur sowohl der Deckschicht als auch der Verästelungen der Stützstruktur an den Außenseiten eine Haut und im Inneren eine poröse Struktur besitzen, wobei die Haut sowohl der Deckschichten als auch der Verästelungen in der Stützstruktur bei Untersuchung mittels Rasterelektronenmikroskopie glatt, gleichmäßig und ebenmäßig erscheint. Letzteres ist in Fig. 5 und Fig. 6 anhand von Ausschnitten der Membranoberfläche auf der

Walzenseite und auf der Luftseite bei einer 500 fachen Vergrößerung verdeutlicht.

Die Membran des Beispiels 1 wies über ihre Dicke eine Struktur auf, wie sie in der REM-Aufnahme der Figur 7 dargestellt ist. Bei der hier gezeigten Vergrößerung von ca. 1600 ist deutlich zu erkennen, dass die überwiegende Mehrzahl der die Struktur aufbauenden Verästelungen an ihrer jeweils dünnsten Stelle einen Durchmesser von mehr als 0,5 $\mu$m aufweisen. Aus den Figuren 3 bis 6 ist auch ersichtlich, dass die Öffnungen bzw. Poren in den Oberflächen der Membran des Beispiels 1 eine Insel-im-Meer-Struktur (island-sea-structure) aufweisen, bei der im Mittel näherungsweise ovale oder kreisförmige Öffnungen in den Oberflächen von der Deckschicht umgeben sind, die Deckschichten also jeweils die kontinuierliche Phase in Form von die Öffnungen bzw. Poren umschließenden Stegen ausbilden, die die Öffnungen umschließt.

Beispiel 2:

Es wurde wie in Beispiel 1 vorgegangen, wobei jedoch eine Fällbadtemperatur von 20°C eingestellt wurde.

Die Membran hatte eine Dicke von ca. 100 $\mu$m und war ebenfalls permanent hydrophil und spontan mit Wasser benetzbar. Sie hatte eine mittels Blaspunktmethode bestimmte maximale Trennpore von 8,3 $\mu$m und wies einen Transmembranfluss von ca. 2080 ml/(cm$^2$·min·bar) sowie eine Porosität von ca. 83 Vol.-% auf. Die Membran hatte eine mittlere Bruchfestigkeit von 740 cN/mm$^2$ in Längsrichtung (Produktionsrichtung) und von 730 cN/mm$^2$ in Querrichtung. Die mittlere Bruchdehnung betrug in Längsrichtung 25% und in Querrichtung 43%. Der mittlere Durchmesser der Poren in der Oberfläche, die beim Gießen des Films der Gießwalze zugewandt war (Walzenseite), betrug 10,5 $\mu$m, derjenige der Poren in der Oberfläche, die beim Gießen des Films zunächst der Luft zugewandt war (Luftseite), betrug 8,2 $\mu$m. Der über bildanalytische Messverfahren ermittelte Anteil der Poren in der Oberfläche auf der Walzenseite betrug 36,2% und auf der Luftseite 6,8 %. Im Test hielt die Membran problemlos eine Wassersäule von mindestens einem Meter.

Die Membranstruktur ähnelt derjenigen der Membran des Beispiels 1. Anhand der Figuren 8 und 9, die einen Querschnitt der Membran des Beispiels 2 in 250-facher bzw. 500-facher Vergrößerung zeigen, sind Deckschichten an beiden Oberflächen der Membran erkennbar. Die dazwischenliegende Stützschicht weist einen ersten, von der Walzenseite (rechts in der in Fig. 8 bzw. 9 gezeigten REM-Aufnahme) ausgehenden Bereich mit im wesentlichen isotroper Porenstruktur auf, der sich über ca. 70 % der Stützschicht erstreckt. In einem sich an den ersten Bereich der Stützschicht in Richtung auf die Luftseite der Membran erstreckenden zweiten Bereich erscheint die Porenstruktur grobporiger als im ersten Bereich.

Auch anhand der Figuren 8 und 9 ist deutlich zu erkennen, dass die Deckschichten und die Verästelungen in ihrem Inneren eine poröse Struktur aufweisen und an ihrer Außenseite eine glatte, ebenmäßige Haut. Die Figuren 10 und 11 stellen rasterelektronenmikroskopische Aufnahmen der Oberflächen der Membran des Beispiels 2 dar (Fig. 10: Walzenseite; Fig. 11: Luftseite). Auch diese Oberflächen weisen eine deutlich eine Insel-im-Meer-Struktur (island-sea-structure) auf, bei der näherungsweise ovale oder kreisförmige Öffnungen in den Oberflächen von der Deckschicht umgeben sind. Fig. 12 zeigt in einer ca. 1600-fachen Vergrößerung eine REM-Aufnahme des Querschnitts der Membran des Beispiels 2. Es ist deutlich erkennbar, dass nahezu alle der Verästelungen bzw. Stege an ihrer dünnsten Stelle einen Durchmesser von mindestens 0,5 $\mu$m aufweisen.

Beispiel 3:

Es wurde die Gießlösung aus Beispiel 1 verwendet. Die Gießlösung wurde mittels eines auf 67°C temperierten Gießkastens auf einer auf 66°C temperierten Metall-Gießwalze zu einem Film mit einer Dicke von ca. 140 $\mu$m ausgegossen. Der auf der Gießwalze befindliche Film wurde durch eine Klimazone geführt und während 16 s mit einem Klima von 59°C und 72% rel. Luftfeuchte (Taupunkt ca. 45°C) beaufschlagt, bevor er in ein Koagulationsbad mit auf 20°C temperiertem Wasser eingeführt wurde. Nach einer Verweilzeit von 16 s zur Ausbildung der Membranstruktur wurde der Film mittels einer Abzugswalze von der Gießwalze abgezogen. In nachfolgenden Waschbädern wurde die Membran bei stufenweise auf 90°C ansteigenden Temperaturen in Wasser fixiert und das Lösemittel zusammen mit Großteilen des PVP extrahiert. Die Trocknung der Membran erfolgte mittels eines Walzentrockners.

Die so hergestellte Membran war permanent hydrophil und spontan mit Wasser benetzbar und hatte eine Dicke von ca. 110 $\mu$m sowie eine mittels Blaspunktmethode bestimmte maximale Trennpore von 9,0 $\mu$m. Sie wies einen Transmembranfluss von ca. 2000 ml/(cm$^2$·min·bar) auf und hatte eine Porosität von ca. 81 Vol.-%. Für die Membran wurde eine Bruchfestigkeit von 720 cN/mm$^2$ in Längsrichtung (Produktionsrichtung) und von 700 cN/mm$^2$ in Querrichtung gemessen. Die mittlere Bruchdehnung betrug in Längsrichtung 29% und in Querrichtung 44%. Im Test hielt die Membran problemlos eine Wassersäule von mindestens einem Meter.

Hinsichtlich ihrer Struktur, d.h. auch hinsichtlich der die Struktur aufbauenden Verästelungen ähnelte die Membran des Beispiels 3 der Membran des Beispiels 1 bzw. des Beispiels 2. Im Querschnitt wies die Membran des Beispiels 3 anhand von REM-Aufnahmen erkennbare Deckschichten an beiden Oberflächen auf. Die dazwischenliegende Stützschicht weist gemäß Fig. 13 und Fig. 14 einen ersten, von der Walzenseite (rechts in den in Fig. 13 bzw. 14 gezeigten

REM-Aufnahmen) ausgehenden Bereich mit im wesentlichen isotroper Porenstruktur auf, der sich über ca. 60 % der Stützschicht erstreckt. In einem sich an diesen ersten Bereich der Stützschicht in Richtung auf die Luftseite der Membran erstreckenden zweiten Bereich erscheint die Porenstruktur grobporiger als im ersten isotropen Bereich. Die Figuren 15 und 16 stellen Rasterelektronenmikroskop-Aufnahmen der Oberflächen der Membran des Beispiels 3 dar (Fig. 15: Walzenseite; Fig. 16: Luftseite). Auch diese Oberflächen weisen eine deutlich eine Insel-im-Meer-Struktur (island-sea-structure) auf, bei der näherungsweise ovale oder kreisförmige Öffnungen in den Oberflächen von der Deckschicht umgeben sind.

Vergleichsbeispiel 1:

Es wurde wie in Beispiel 1 vorgegangen. Abweichend von Beispiel 1 lag die Gießwalzentemperatur bei 60°C und die Fällbadtemperatur bei 55°C. Darüber hinaus lag die Temperatur in der klimatisierten Zone bei 36,5°C und die relative Luftfeuchtigkeit bei ca. 95% (Taupunkt ca. 33°C). Des Weiteren wurde das Verfahren so durchgeführt, dass sich in der Klimazone und im Fällbad jeweils eine Verweilzeit von ca. 18,5 s ergab.

Die so hergestellte, ca. 95 μm dicke Membran hatte eine vergleichsweise feinporige, schwammartige Porenstruktur mit feinen Verästelungen. Die mittels Blaspunktmethode bestimmte maximale Trennpore ergab sich zu 2,4 μm. Die Membran wies einen Transmembranfluss von ca. 474 ml/($cm^2$·min·bar) und damit einen zu geringen Transmembranfluss auf.

In dem in Figur 17 dargestellten Querschnitt zeigt die Membran des Vergleichsbeispiels 1 über die Wanddicke gesehen einen asymmetrischen Aufbau. Im Wandinneren, nahe der Wandmitte, liegt ein Bereich mit minimaler Porengröße. Ausgehend von diesem Bereich mit minimaler Porengröße nimmt die Größe der Poren zu beiden Oberflächen hin zu, d.h. zu beiden Seiten des Bereichs mit minimaler Porengröße befindet sich ein asymmetrischer Bereich. Die Figuren 18 und 19 zeigen die Oberflächen der Membran des Vergleichsbeispiels 1 dar (Fig. 18: Walzenseite; Fig. 19: Luftseite).

Vergleichsbeispiel 2:

Es wurde wie in Beispiel 1 vorgegangen. Abweichend von Beispiel 1 lagen die Gießwalzentemperatur und die Fällbadtemperatur bei 27°C. Darüber hinaus lag die Temperatur in der klimatisierten Zone bei 40°C und die relative Luftfeuchtigkeit bei ca. 78% (Taupunkt ca. 35°C). Des weiteren wurde das Verfahren so durchgeführt, dass sich in der Klimazone und im Fällbad jeweils eine Verweilzeit von ca. 12,5 s ergab.

Die so hergestellte Membran hatte ebenfalls eine feinporige, schwammartige Porenstruktur. Die mittels Blaspunktmethode bestimmte maximale Trennpore ergab sich zu ca. 0,5 μm. Die Membran wies nur einen Transmembranfluss von ca. 30 ml/($cm^2$·min·bar), der damit deutlich zu gering war.

Der in Figur 20 dargestellte Querschnitt der Membran des Vergleichsbeispiels 1 zeigt über der Wanddicke eine asymmetrische Porenstruktur mit einem sich im Wandinneren nahe der Wandmitte befindenden Bereich mit minimaler Porengröße. Ausgehend von diesem Bereich mit minimaler Porengröße nimmt die Größe der Poren zu beiden Oberflächen hin zu, d.h. zu beiden Seiten des Bereichs mit minimaler Porengröße befindet sich ein asymmetrischer Bereich. Die Figuren 21 und 22 stellen rasterelektronenmikroskopische Aufnahmen der Oberflächen der Membran des Vergleichsbeispiels 2 dar (Fig. 21: Walzenseite; Fig. 22: Luftseite).

Vergleichsbeispiele 3a und 3b:

Es wurde wie in Beispiel 1 vorgegangen. Abweichend von Beispiel 1 lagen Gießwalzentemperatur und Fällbadtemperatur bei 79°C bzw. 75°C. Die Temperatur in der klimatisierten Zone wurde auf 50°C und die relative Luftfeuchtigkeit auf ca. 75% (Taupunkt ca. 42°C) eingestellt. Des weiteren wurde das Verfahren so durchgeführt, dass sich in der Klimazone und im Fällbad jeweils eine Verweilzeit von ca. 26 s ergab. Darüber hinaus wurde abweichend von Beispiel 1 auf der Gießwalze ein Film mit einer Dicke von ca. 370 μm (Vergleichsbeispiel 3a) bzw. von ca. 420 μm (Vergleichsbeispiel 3b) ausgegossen, woraus Membranen mit einer Dicke von 300 μm bzw. 350 μm erhalten wurden.

Die Eigenschaften der Membranen der Vergleichsbeispiele 3a und 3b sind in der Tabelle 1 aufgeführt.

Tabelle 1:

| Membran aus | Dicke μm | TMF ml/($cm^2$·min·bar) | maximale Trennpore μm |
|---|---|---|---|
| Vergleichsbeispiel 3a | 300 | 870 | 4,8 |
| Vergleichsbeispiel 3b | 350 | 270 | 4,8 |

Der in den Figuren 23 und 24 dargestellte Querschnitt der Membran des Vergleichsbeispiels 3b zeigt über der Wanddicke eine deutlich asymmetrische Porenstruktur mit einem an der Walzenseite (rechts in den in Fig. 23 und Fig. 24 gezeigten REM-Aufnahmen) zunächst feinporigeren Struktur, die zum Wandinneren in eine sehr offenporige Struktur übergeht. Zur Luftseite hin (links in den in Fig. 23 und Fig. 24 gezeigten REM-Aufnahmen) geht die Struktur in einen sich über ca. 25% der Wanddicke erstreckenden feinporigeren Bereich über.

Die Figuren 25 und 26 stellen rasterelektronenmikroskopische Aufnahmen der Oberflächen der Membran des Vergleichsbeispiels 3b dar (Fig. 25: Walzenseite; Fig. 26: Luftseite). An der Walzenseite zeigt die Membran des Vergleichsbeispiels 3b eine z.T. netzwerkartige und fibrilläre Struktur mit überwiegend dünnen, fibrillenartigen Begrenzungen zwischen unregelmäßig geformten Poren.

Vergleichsbeispiel 4:

Es wurde nach der Vorgehensweise gemäß Beispiel 1 eine homogene Lösung aus 13,95 Gew.-% Polyethersulfon (PES, Typ Ultrason E6020, Fa. BASF), 1,04 Gew.-% sulfoniertem Polyethersulfon (SPES) mit einem Sulfonierungsgrad von 5% und 11,25 Gew.-% Polyvinylpyrrolidon (PVP, Typ K30, Fa. ISP) in einem Lösemittelsystem mit 41,48 Gew.-% $\gamma$-Butyrolacton, 13,83 Gew.-% $\varepsilon$-Caprolactam und 18,45 Gew.-% Polyethylenglykol PEG 200, jeweils bezogen auf das Geweicht der Gesamtlösung, hergestellt. Die fertige Lösung hatte bei einer Messtemperatur von 40°C eine Viskosität von 3,7 Pa s.

Die fertige Gießlösung wurde mittels eines auf 45°C temperierten Gießkastens auf eine auf 75°C temperierte Metall-Gießwalze zu einem Film ausgegossen. Der auf der Gießwalze befindliche Film wurde durch eine Klimazone geführt und während ca. 26 s mit einem Klima von 50°C und 74% rel. Luftfeuchte (Taupunkt ca. 47°C) beaufschlagt, bevor er in ein Koagulationsbad mit auf 69,5°C temperiertem Wasser eingeführt wurde. Nach einer Verweilzeit von 26 s zur Ausbildung der Membranstruktur wurde der Film mittels einer Abzugswalze von der Gießwalze abgezogen. In nachfolgenden Waschbädern wurde die Membran in Wasser fixiert und das Lösemittel zusammen mit Großteilen des PVP extrahiert. Die Trocknung der Membran erfolgte mittels eines Walzentrockners.

Es wurde eine Membran mit einer Dicke von ca. 280 $\mu$m hergestellt. Die Membran hatte eine mittels Blaspunktmethode bestimmte maximale Trennpore von 3,9 $\mu$m und wies einen Transmembranfluss von ca. 1250 ml/(cm$^2$·min·bar).

Ausweislich der REM-Aufnahmen des Membranwandquerschnitts und der Oberflächen gemäß den Figuren 27 bis 29 hatte die Membran dieses Vergleichsbeispiels 4 eine sich über die gesamte Membranwand erstreckende ausgeprägt asymmetrische Struktur, wobei sich die Durchmesser der Poren in den Oberflächen der Membran im Mittel um mehr als den Faktor 2 unterschieden.

Vergleichsbeispiel 5

Es wurde wie in Vergleichsbeispiel 4 vorgegangen. Abweichend von Vergleichsbeispiel 4 wurde auf eine auf 85°C temperierten Gießwalze ein Film ausgegossen, der nach Durchlaufen der Klimazone in ein Koagulationsbad mit auf 73°C temperiertem Wasser eingeführt wurde, um die Membranstruktur auszubilden.

Es wurde eine Membran mit einer Dicke von ca. 340 $\mu$m hergestellt. Die Membran hatte eine maximale Trennpore von 2,5 $\mu$m und wies einen Transmembranfluss von ca. 630 ml/(cm$^2$·min·bar). Die Struktur der Membran des Vergleichsbeispiels 5 ähnelte derjenigen der Membran des Vergleichsbeispiels 4.

Vergleichsbeispiel 6:

Es wurde nach der Vorgehensweise gemäß Beispiel 1 eine homogene Lösung aus 13,84 Gew.-% Polyethersulfon (PES, Typ Ultrason E6020, Fa. BASF), 1,04 Gew.-% sulfoniertem Polyethersulfon (SPES) mit einem Sulfonierungsgrad von 5% und 11,16 Gew.-% Polyvinylpyrrolidon (PVP, Typ K30, Fa. ISP) in einem Lösemittelsystem mit 41,15 Gew.-% $\gamma$-Butyrolacton, 13,72 Gew.-% $\varepsilon$-Caprolactam, 18,29 Gew.-% Polyethylenglykol PEG 200 und 0,80 Gew.-% Wasser, jeweils bezogen auf das Geweicht der Gesamtlösung, hergestellt. Die fertige Lösung hatte eine Viskosität von 3,7 Pa s bei einer Messtemperatur von 40°C.

Die fertige Gießlösung wurde mittels eines auf 45°C temperierten Gießkastens auf eine auf 64°C temperierte Metall-Gießwalze zu einem Film mit einer Dicke von ca. 170 $\mu$m ausgegossen. Der auf der Gießwalze befindliche Film wurde durch eine Klimazone geführt und während ca. 10 s mit einem Klima von 46°C und 42% rel. Luftfeuchte (Taupunkt ca. 29°C) beaufschlagt, bevor er in ein Koagulationsbad mit auf 64°C temperiertem Wasser eingeführt wurde. Nach einer Verweilzeit von 10 s zur Ausbildung der Membranstruktur wurde der Film mittels einer Abzugswalze von der Gießwalze abgezogen. In nachfolgenden Waschbädern wurde die Membran in Wasser fixiert extrahiert. Die Trocknung der Membran erfolgte mittels eines Walzentrockners.

Die Membran hatte eine Dicke von ca. 140 $\mu$m, eine mittels Blaspunktmethode bestimmte maximale Trennpore von 0,8 $\mu$m sowie einen Transmembranfluss von ca. 220 ml/(cm$^2$·min·bar). Die Membran wies eine über die gesamte

Membranwand sich erstreckende asymmetrische, vergleichsweise feinporige schwammartige Porenstruktur mit überwiegend feinen, dünnen Verästelungen auf. Anhand von REM-Aufnahmen ist ersichtlich, dass sich im Wandinneren eine Schicht mit kleinsten Poren befindet und die Porengröße ausgehend von dieser Schicht in Richtung auf die Oberflächen hin zunimmt (Fig. 30).

**Patentansprüche**

1. Hydrophile großporige Filtrationsmembran in Gestalt einer Flachmembran auf Basis eines filmbildenden hydrophoben ersten Polymers aus der Gruppe der aromatischen Sulfonpolymere sowie eines hydrophilen zweiten Polymers, wobei die Membran aus einer ersten und einer zweiten Oberfläche und einer sich zwischen den Oberflächen erstreckende Stützschicht mit einer dreidimensionalen schwammartigen Netzwerkstruktur besteht,

    - wobei die Stützschicht an ihrer der ersten Oberfläche zugewandten Seite eine erste Deckschicht und an ihrer der zweiten Oberfläche zugewandten Seite eine zweite Deckschicht aufweist, die integral mit der Stützschicht ausgebildet sind, und
    - wobei die erste und die zweite Oberfläche näherungsweise ovale oder kreisförmige Öffnungen aufweisen, die die erste bzw. zweite Deckschicht durchdringen und mit der Stützschicht in Verbindung stehen, wobei der mittlere Durchmesser der Öffnungen in der zweiten Oberfläche 0,5 bis 1,5 mal so groß wie der mittlere Durchmesser der Öffnungen in der ersten Oberfläche ist und unter der Bedingung, dass der mittlere Durchmesser der Öffnungen in der ersten Oberfläche und der mittlere Durchmesser der Öffnungen in der zweiten Oberfläche sich um weniger als den Faktor 2 unterscheiden,
    - wobei die dreidimensionale Netzwerkstruktur der Stützschicht aus dicken Verästelungen und einem Porensystem mit durchgehenden Poren aufgebaut ist und mindestens 70 % der Verästelungen an ihrer dünnsten Stelle einen Durchmesser durch Bestimmung anhand von Rasterelektronenmikroskop-Aufnahmen von Querschnitten der Membranen in einer Vergrößerung von mindestens 1000 von mindestens 0,5 μm haben, wobei die dreidimensionale Netzwerkstruktur keine kavernenartigen Poren, die auch als Fingerporen bezeichnet werden, enthält;
    - wobei die Poren in der Stützschicht größer sind als die Öffnungen in den Oberflächen;
    - wobei die Filtrationsmembran eine Dicke im Bereich zwischen 50 und 150 μm aufweist; und
    - der mittlere Durchmesser der Öffnungen in der zweiten Oberfläche durch Bestimmung anhand von Rasterelektronmikroscop-Aufnahmen im Bereich von 2 bis 20 μm liegt.

2. Großporige Filtrationsmembran nach Anspruch 1, wobei der poröse Flächenanteil der ersten Oberfläche mindestens 20 % beträgt.

3. Großporige Filtrationsmembran nach Anspruch 1 oder 2, wobei der poröse Flächenanteil der ersten Oberfläche kleiner als 60 % ist.

4. Großporige Filtrationsmembran nach einem oder mehreren der Ansprüche 1 bis 3, wobei die erste bzw. die zweite Deckschicht eine Dicke gemäß Bestimmung anhand von Rasterelektronenmikroskop-Aufnahmen der Membranen im Bereich von 3 bis 10 μm aufweist.

5. Großporige Filtrationsmembran nach einem oder mehreren der Ansprüche 1 bis 4, wobei der Durchmesser der Poren in der Stützschicht, ausgehend von der ersten Deckschicht in Richtung auf die zweite Deckschicht über mindestens 50 % der Dicke der Stützschicht in einem ersten Bereich der Stützschicht im wesentlichen konstant ist.

6. Großporige Filtrationsmembran nach einem oder mehreren der Ansprüche 1 bis 5, wobei sie eine Volumenporosität gemäß der Bestimmung im Experimentalteil der Beschreibung im Bereich von 65 bis 85 Vol.-% aufweist.

7. Großporige Filtrationsmembran nach einem oder mehreren der Ansprüche 1 bis 6, wobei sie einen mittels der Blaspunktmethode ermittelten Durchmesser der maximalen Trennpore $d_{max}$ im Bereich von 5 bis 10 μm aufweist.

8. Großporige Filtrationsmembran nach einem oder mehreren der Ansprüche 1 bis 7, wobei die Stützschicht Poren mit einem Durchmesser von mindestens 1/10 der Membrandicke aufweist.

9. Großporige Filtrationsmembran nach einem oder mehreren der Ansprüche 1 bis 8, wobei sie einen Transmembranfluss TMF für Wasser gemäß der Bestimmung bei 25 °C im Experimentalteil im Bereich von 700 bis 4000

ml/(cm$^2$·min·bar) aufweist.

**10.** Großporige Filtrationsmembran nach einem oder mehreren der Ansprüche 1 bis 9, wobei sie eine auf ihren Querschnitt bezogene Zugfestigkeit gemäß der Bestimmung im Experimentalteil von mindestens 500 cN/mm$^2$ aufweist.

**11.** Großporige Filtrationsmembran nach einem oder mehreren der Ansprüche 1 bis 10, wobei das aromatische Sulfonpolymere ein Polysulfon oder Polyethersulfon ist.

**12.** Großporige Filtrationsmembran nach einem oder mehreren der Ansprüche 1 bis 11, wobei das hydrophile Polymer ein Polyvinylpyrrolidon, ein Polyethylenglykol, ein Polyvinylalkohol, ein Polyglykolmonoester, ein Polysorbitat, ein Polyacrylat, eine Carboxylmethylcellulose, eine Polyacrylsäure, oder eine Modifikation oder ein Copolymer dieser Polymere ist.

**13.** Infusionsbesteck zur Verabreichung von Infusionslösungen, umfassend eine Tropfkammer und einen mit der Tropfkammer verbundenen Infusionsschlauch, wobei im Bereich des Ausgangs der Tropfkammer in den Infusionsschlauch eine Membran gemäß einem oder mehreren der Ansprüche 1 bis 12 eingebracht ist.

## Claims

**1.** A hydrophilic, macroporous filtration membrane in the form of a flat membrane based on a film-forming hydrophobic first polymer from the group of aromatic sulfone polymers, and a hydrophilic second polymer, wherein the membrane consists of a first and a second surface, and a supporting layer extending between the surfaces and having a three-dimensional sponge-like network structure,

- wherein the supporting layer has a first cover layer on the side thereof facing the first surface and a second cover layer on the side thereof facing the second surface, which cover layers are formed integrally with the supporting layer, and
- wherein the first and second surfaces have approximately oval or circular openings which penetrate the first and second cover layers, respectively, and are connected to the supporting layer, wherein the average diameter of the openings in the second surface is 0.5 to 1.5 times the size of the average diameter of the openings in the first surface and on condition that the average diameter of the openings in the first surface and the average diameter of the openings in the second surface differ by less than a factor of 2,
- wherein the three-dimensional network structure of the supporting layer is made up of thick branches and a pore system with continuous pores, and at least 70% of the branches have a diameter at the thinnest point thereof, determined using scanning electron micrographs of cross-sections of the membranes at a magnification of at least 1000, of at least 0.5 μm, wherein the three-dimensional network structure does not contain any cavern-like pores, also referred to as finger pores;
- wherein the pores in the supporting layer are larger than the openings in the surfaces;
- wherein the filtration membrane has a thickness in the range between 50 and 150 μm; and
- the average diameter of the openings in the second surface, determined using scanning electron micrographs, is in the range from 2 to 20 μm.

**2.** The macroporous filtration membrane according to claim 1, wherein the porous proportion of the area of the first surface is at least 20%.

**3.** The macroporous filtration membrane according to claim 1 or 2, wherein the porous proportion of the area of the first surface is less than 60%.

**4.** The macroporous filtration membrane according to one or more of claims 1 to 3, wherein the first or the second cover layer has a thickness, according to determination using scanning electron micrographs of the membranes, in the range from 3 to 10 μm.

**5.** The macroporous filtration membrane according to one or more of claims 1 to 4, wherein the diameter of the pores in the supporting layer, starting from the first cover layer in the direction of the second cover layer, is substantially constant over at least 50% of the thickness of the supporting layer in a first region of the supporting layer.

**6.** The macroporous filtration membrane according to one or more of claims 1 to 5, wherein it has a volume porosity,

according to the determination in the experimental section of the description, of 65 to 85 vol%.

7. The macroporous filtration membrane according to one or more of claims 1 to 6, wherein it has a diameter of the maximum separating pore, $d_{max}$, determined by means of the bubble point method, in the range from 5 to 10 $\mu$m.

8. The macroporous filtration membrane according to one or more of claims 1 to 7, wherein the supporting layer has pores with a diameter of at least 1/10 of the membrane thickness.

9. The macroporous filtration membrane according to one or more of claims 1 to 8, wherein it has a transmembrane flow TMF for water, according to the determination at 25°C in the experimental section, in the range from 700 to 4000 ml/($cm^2$·min·bar).

10. The macroporous filtration membrane according to one or more of claims 1 to 9, wherein it has a tensile strength relative to the cross-section thereof, according to the determination in the experimental section, of at least 500 $cN/mm^2$.

11. The macroporous filtration membrane according to one or more of claims 1 to 10, wherein the aromatic sulfone polymer is a polysulfone or polyether sulfone.

12. The macroporous filtration membrane according to one or more of claims 1 to 11, wherein the hydrophilic polymer is a polyvinylpyrrolidone, a polyethylene glycol, a polyvinyl alcohol, a polyglycol monoester, a polysorbitate, a polyacrylate, a carboxylmethylcellulose, a polyacrylic acid, or a modification or a copolymer of said polymers.

13. An infusion set for administering infusion solutions, comprising a drip chamber and an infusion tube connected to the drip chamber, wherein a membrane according to one or more of claims 1 to 12 is inserted in the infusion tube in the area of the outlet of the drip chamber.

## Revendications

1. Membrane de filtration hydrophile à pores larges sous la forme d'une membrane plate à base d'un premier polymère hydrophobe filmogène choisi dans le groupe des polymères aromatiques sulfonés ainsi que d'un second polymère hydrophile, dans laquelle ladite membrane est constituée d'une première et d'une seconde surface et d'une couche de support s'étendant entre les surfaces et présentant une structure réticulée tridimensionnelle spongieuse,

   - dans laquelle la couche de support comporte, sur son côté orienté vers la première surface, une première couche de recouvrement et, sur son côté orienté vers la seconde surface, une seconde couche de recouvrement, qui sont réalisées d'un seul tenant avec la couche de support, et
   - dans laquelle la première et la seconde surface comprennent des ouvertures approximativement ovales ou circulaires, qui traversent la première ou la seconde couche de recouvrement et sont en communication avec la couche de support, dans laquelle le diamètre moyen des ouvertures dans la seconde surface est de 0,5 et 1,5 fois supérieur au diamètre moyen des ouvertures dans la première surface et, à condition que le diamètre moyen des ouvertures dans la première surface et le diamètre moyen des ouvertures dans la seconde surface diffèrent de moins d'un facteur 2,
   - dans laquelle la structure réticulée tridimensionnelle de la couche de support est constituée de ramifications épaisses et d'un système de pores possédant des pores continus et au moins 70 % des ramifications présentent, dans leur zone la plus mince, un diamètre, tel que déterminé par des images au microscope électronique à balayage des tranches transversales des membranes à un grossissement d'au moins 1 000, d'au moins 0,5 $\mu$m, dans laquelle la structure réticulée tridimensionnelle ne contient pas de pores de type cavité, lesquels sont également appelés pores digitiformes ;
   - dans laquelle les pores de la couche de support sont supérieurs aux ouvertures dans les surfaces ;
   - dans laquelle la membrane de filtration présente une épaisseur dans la plage comprise entre 50 et 150 $\mu$m ; et
   - le diamètre moyen des ouvertures dans la seconde surface, tel que déterminé par des images au microscope électronique à balayage, se trouve dans la plage comprise entre 2 et 20 $\mu$m.

2. Membrane de filtration à pores larges selon la revendication 1, dans laquelle la fraction de surface poreuse de la première surface est d'au moins 20 %.

**3.** Membrane de filtration à pores larges selon la revendication 1 ou 2, dans laquelle la fraction de surface poreuse de la première surface est inférieure à 60 %.

**4.** Membrane de filtration à pores larges selon l'une ou plusieurs des revendications 1 à 3, dans laquelle la première ou la seconde couche de recouvrement présente une épaisseur, telle que déterminée par des images au microscope électronique à balayage des membranes, dans la plage comprise entre 3 à 10 $\mu$m.

**5.** Membrane de filtration à pores larges selon l'une ou plusieurs des revendications 1 à 4, dans laquelle le diamètre des pores dans la couche de support, en partant de la première couche de recouvrement dans la direction de la seconde couche de recouvrement, est essentiellement constant sur au moins 50 % de l'épaisseur de la couche de support dans une première zone de la couche de support.

**6.** Membrane de filtration à pores larges selon l'une ou plusieurs des revendications 1 à 5, dans laquelle ladite membrane présente une porosité volumique, déterminée dans la partie expérimentale de la description, dans la plage comprise entre 65 à 85 % en volume.

**7.** Membrane de filtration à pores larges selon l'une ou plusieurs des revendications 1 à 6, dans laquelle ladite membrane présente un diamètre du pore de séparation maximal $d_{max}$, déterminé selon le procédé de point de bulle, dans la plage comprise entre 5 et 10 $\mu$m.

**8.** Membrane de filtration à pores larges selon l'une ou plusieurs des revendications 1 à 7, dans laquelle la couche de support présente des pores d'un diamètre d'au moins 1/10 de l'épaisseur de la membrane.

**9.** Membrane de filtration à pores larges selon l'une ou plusieurs des revendications 1 à 8, dans laquelle ladite membrane présente un débit transmembranaire TMF pour l'eau déterminé à 25 °C dans la partie expérimentale dans la plage comprise entre 700 et 4 000 ml/($cm^2$ min bar).

**10.** Membrane de filtration à pores larges selon l'une ou plusieurs des revendications 1 à 9, dans laquelle ladite membrane présente une résistance à la traction rapportée à sa section transversale, déterminée dans la partie expérimentale, d'au moins 500 cN/$mm^2$.

**11.** Membrane de filtration à pores larges selon l'une ou plusieurs des revendications 1 à 10, dans laquelle le polymère aromatique sulfoné est une polysulfone ou une polyéthersulfone.

**12.** Membrane de filtration à pores larges selon l'une ou plusieurs des revendications 1 à 11, dans laquelle le polymère hydrophile est une polyvinylpyrrolidone, un polyéthylène glycol, un alcool polyvinylique, un monoester de polyglycol, un polysorbitate, un polyacrylate, une carboxylméthylcellulose, un acide polyacrylique, ou une modification ou un copolymère desdits polymères.

**13.** Jeu de perfusion destiné à l'administration de solutions pour perfusion, comprenant une chambre compte-gouttes et un tuyau de perfusion raccordé à la chambre compte-gouttes, dans lequel une membrane selon l'une ou plusieurs des revendications 1 à 12 est introduite dans le tuyau d'infusion dans une zone d'une sortie de la chambre compte-gouttes.

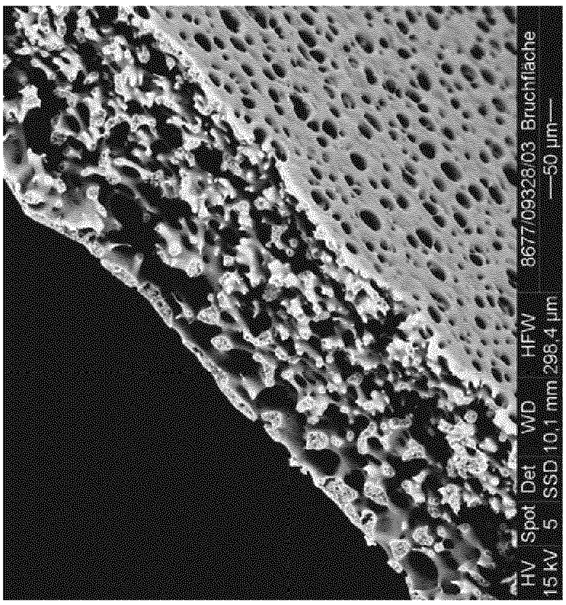


Fig. 1

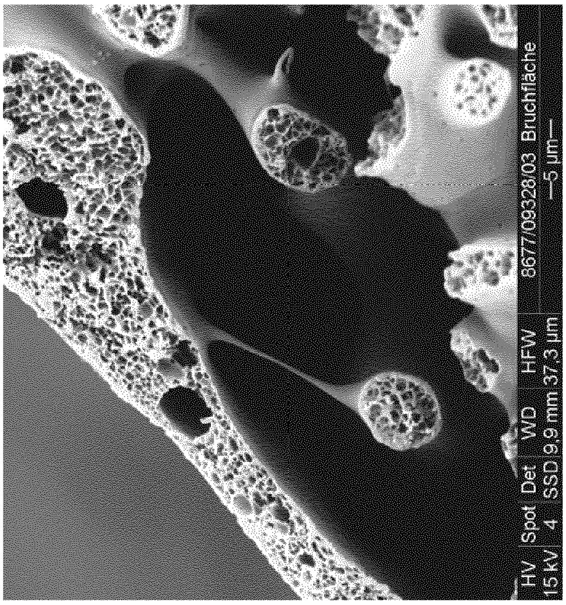


Fig. 2

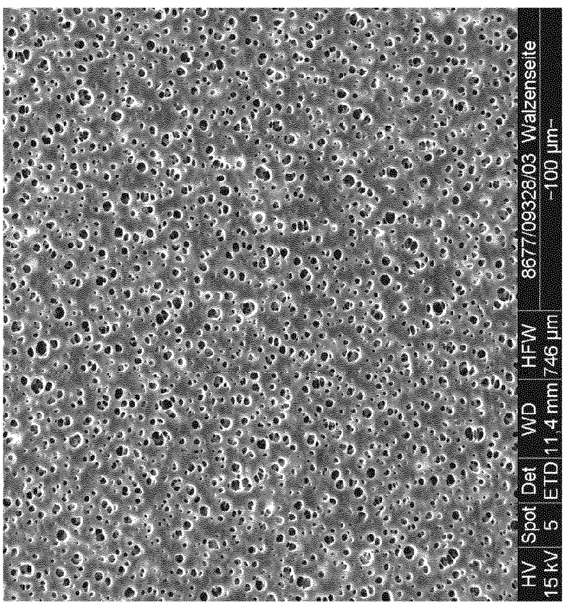


Fig. 3

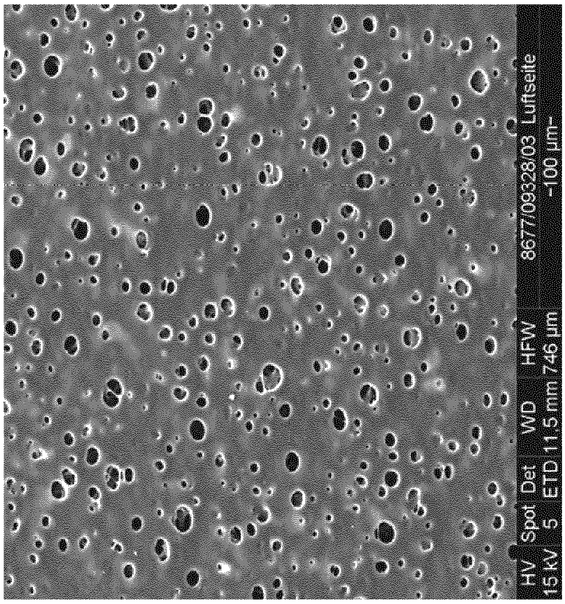


Fig. 4

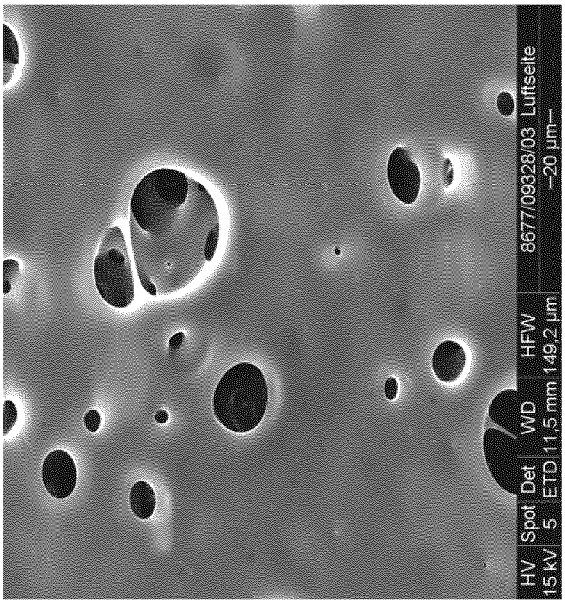
HV Spot Det WD HFW 8677/09328/03 Luftseite
15 kV 5 ETD 11,5 mm 149,2 µm —20 µm—

Fig. 6

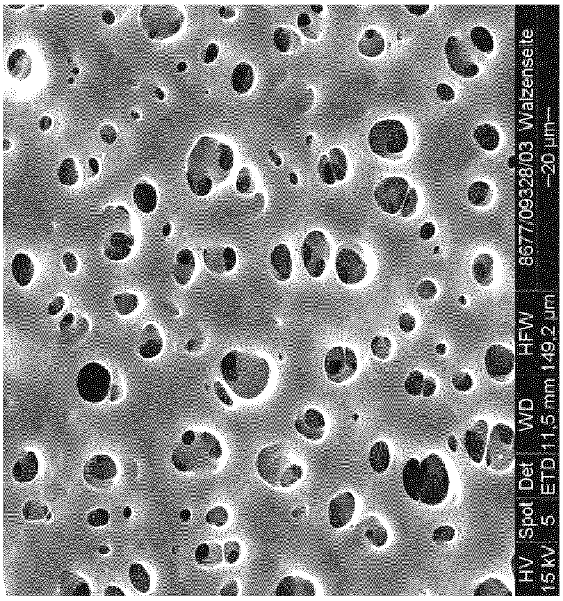
HV Spot Det WD HFW 8677/09328/03 Walzenseite
15 kV 5 ETD 11,5 mm 149,2 µm —20 µm—

Fig. 5

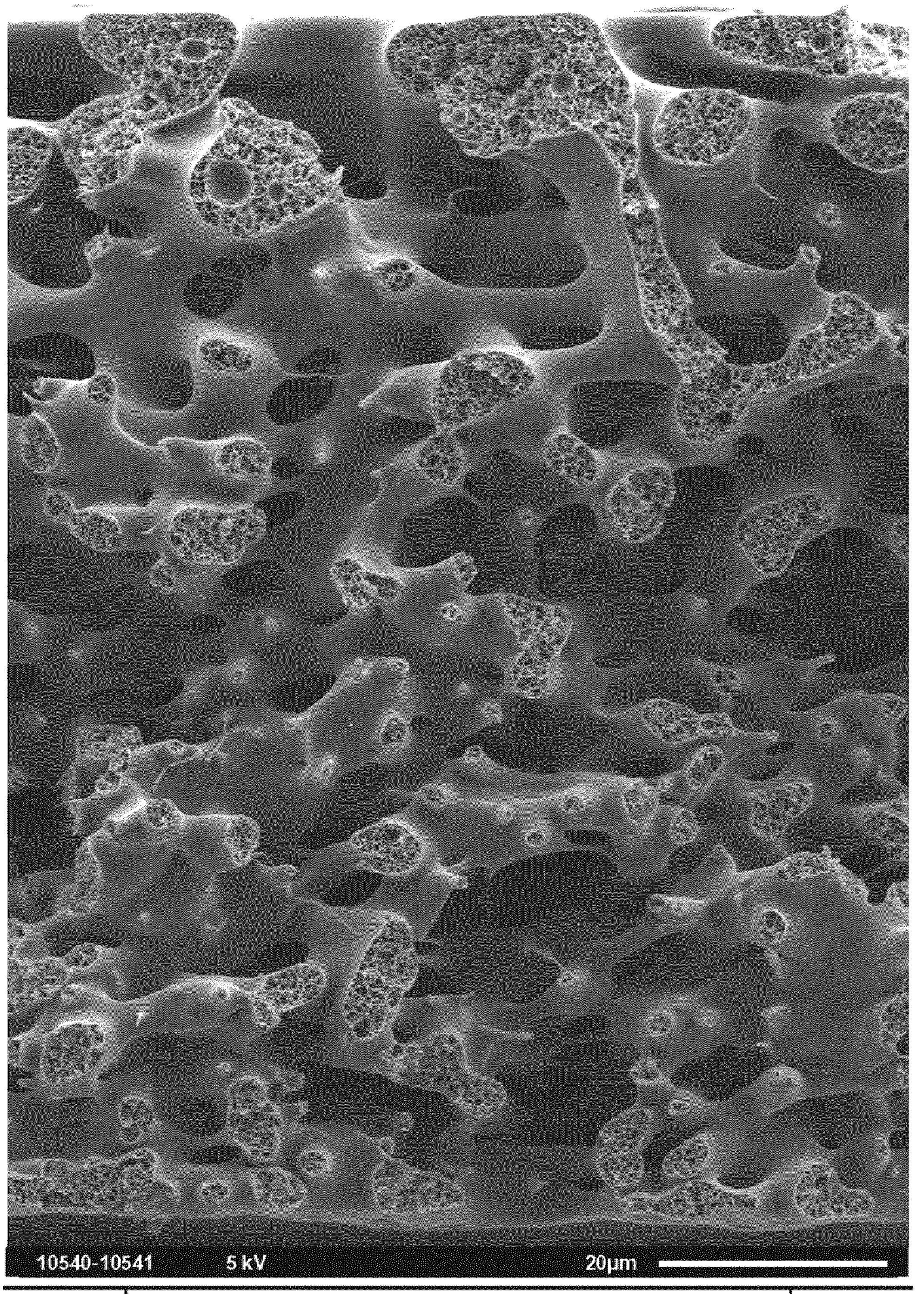


Fig. 7

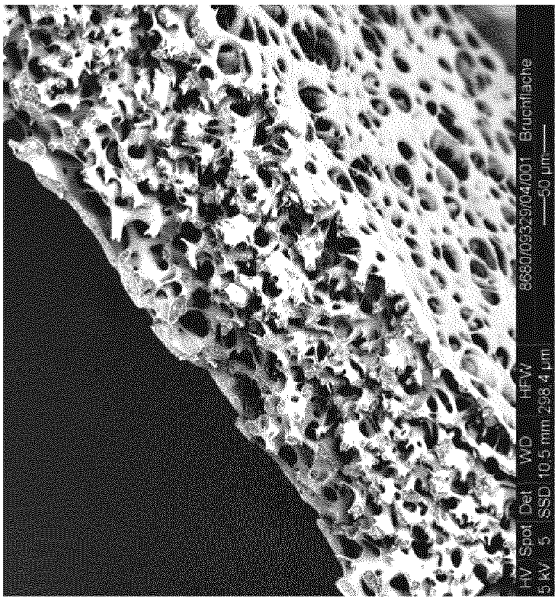


Fig. 8

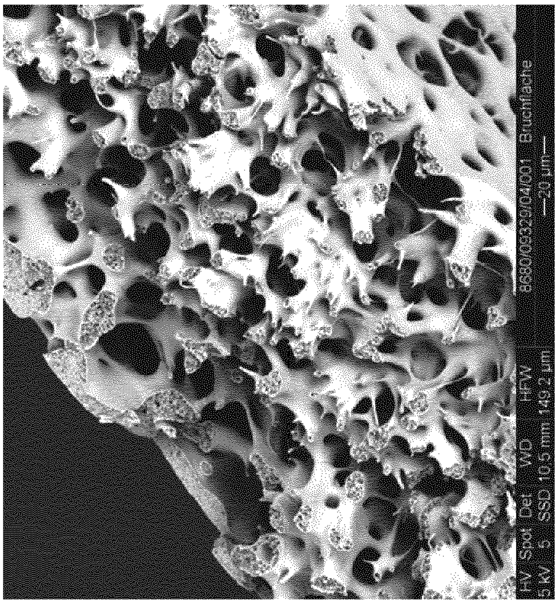


Fig. 9

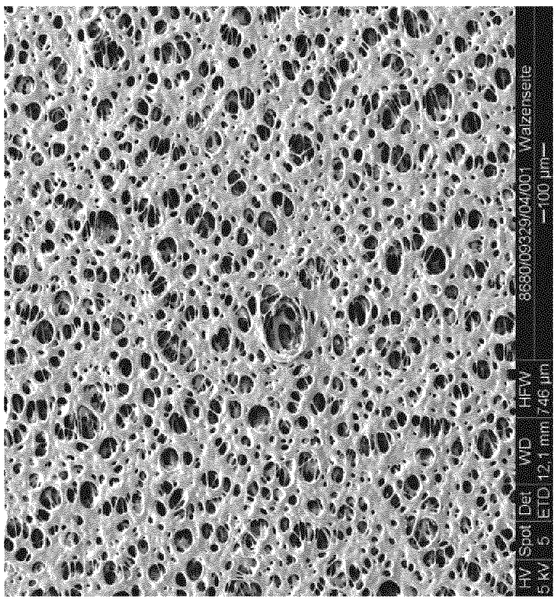


Fig. 10

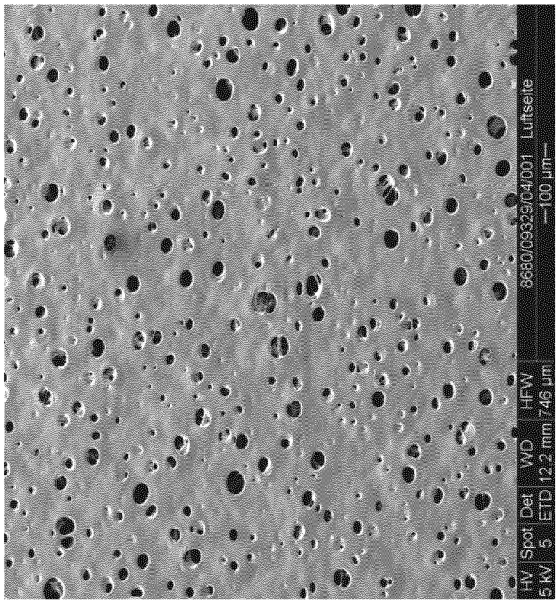


Fig. 11

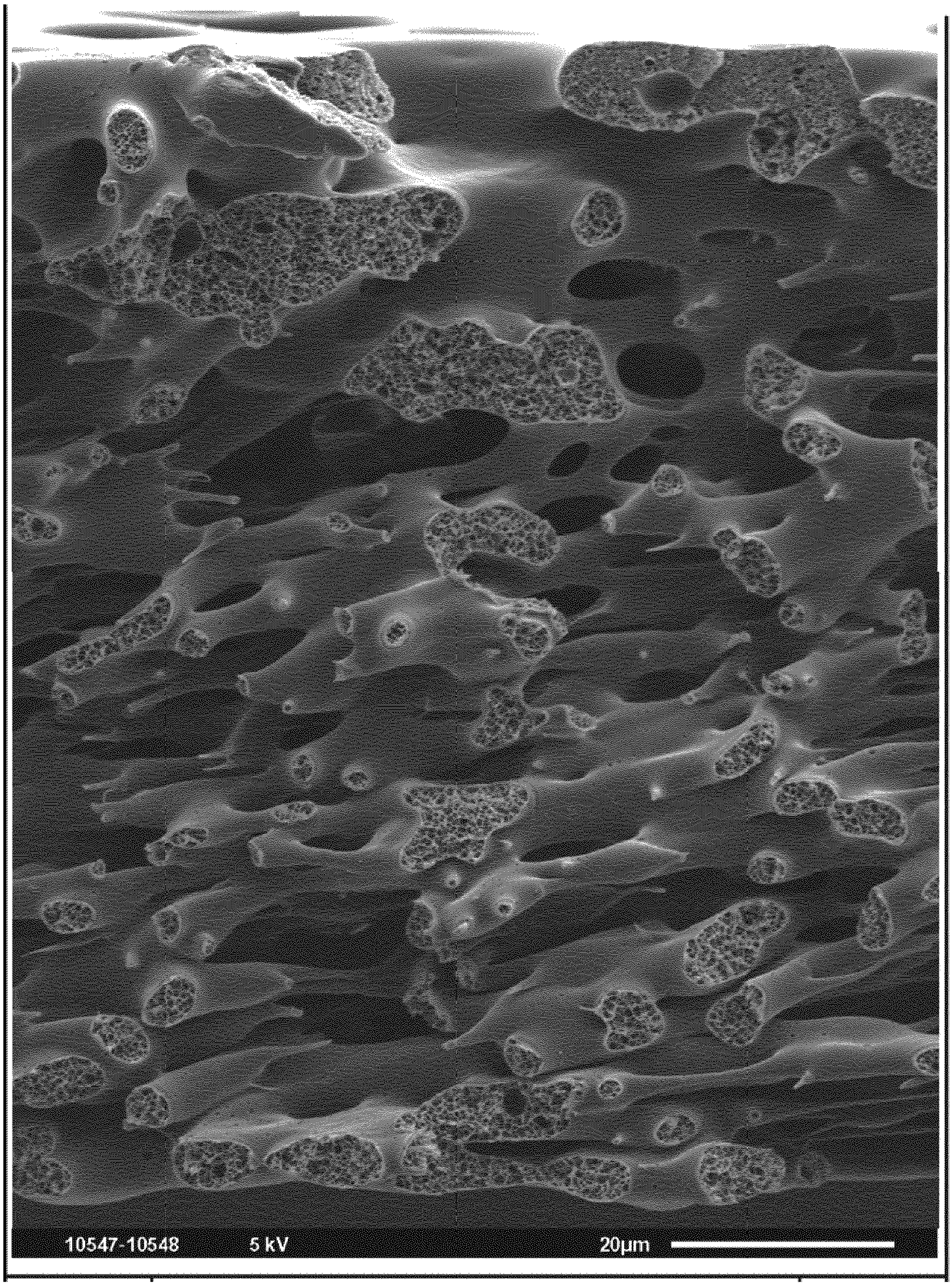


Fig. 12

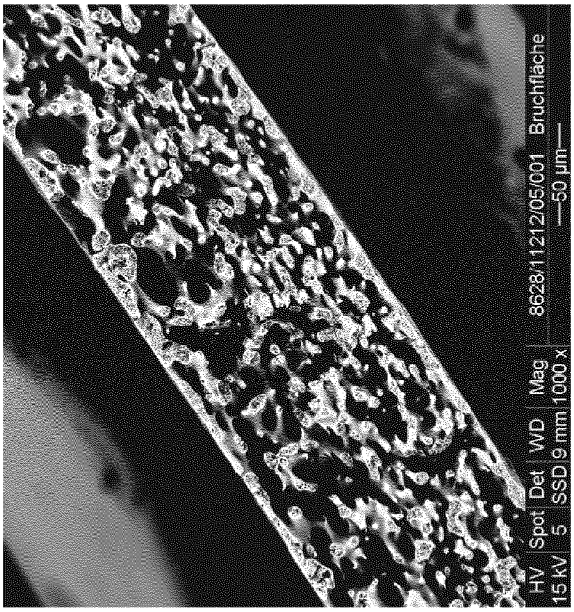


Fig. 13

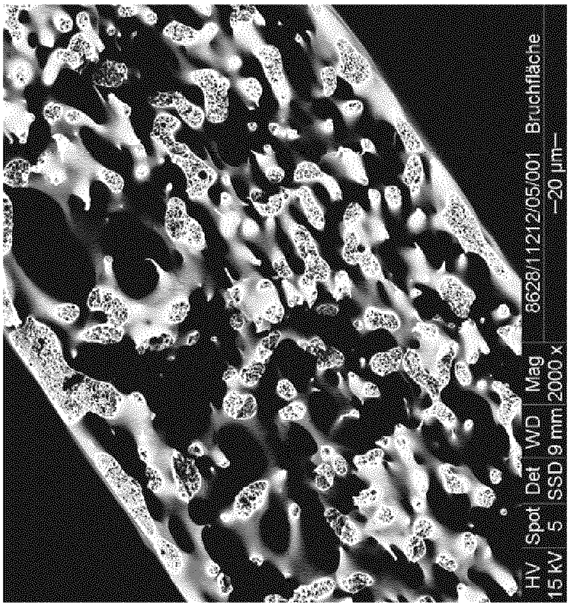


Fig. 14

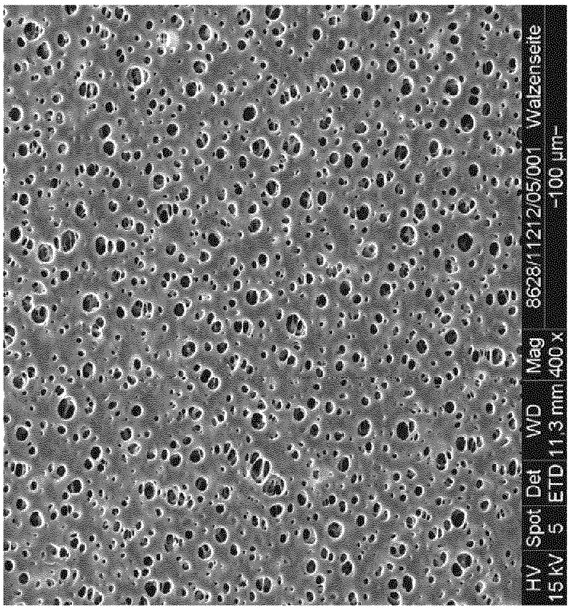


Fig. 15

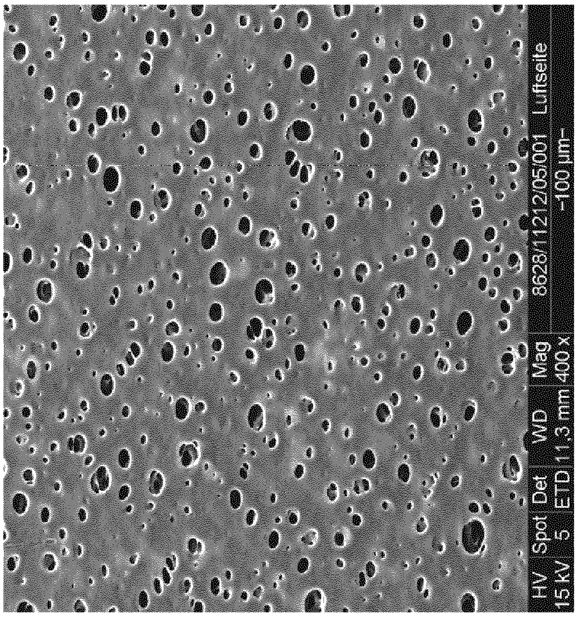


Fig. 16

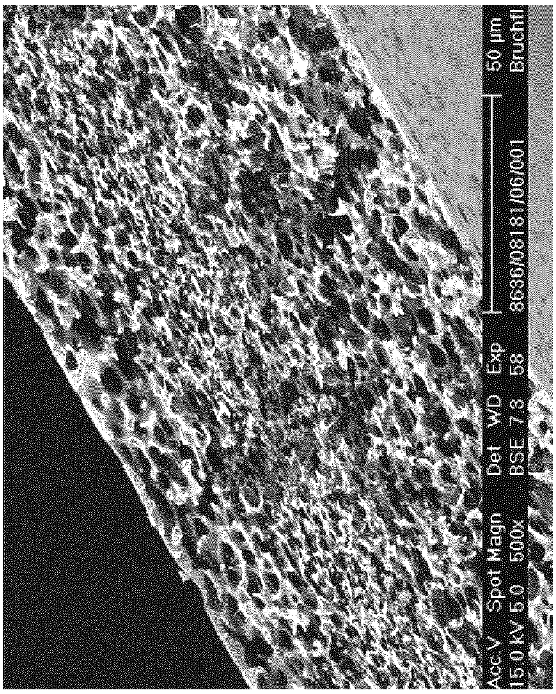


Fig. 17

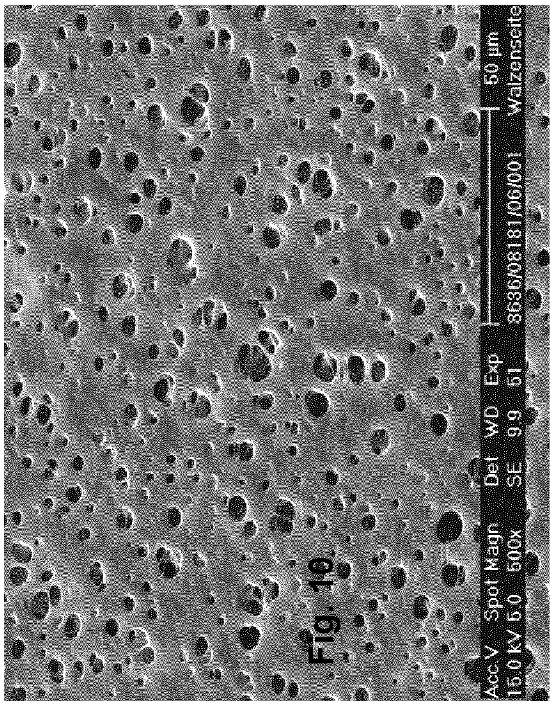


Fig. 18

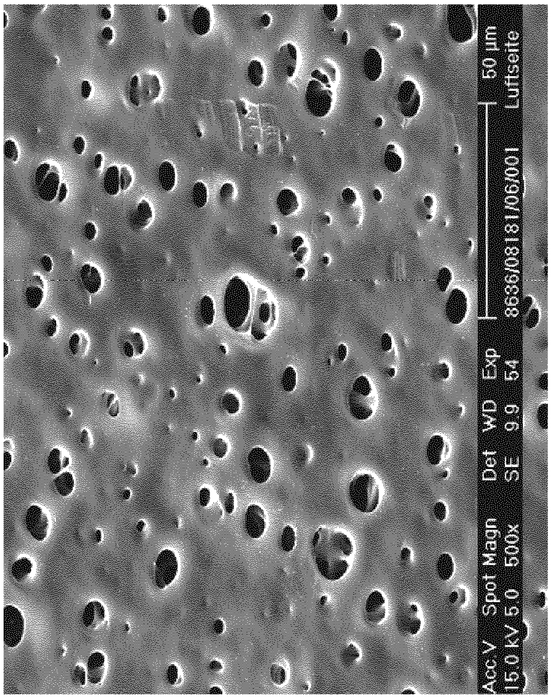


Fig. 19

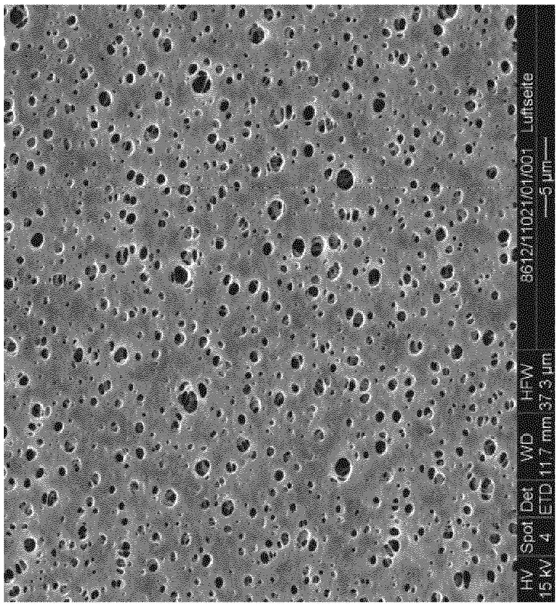


Fig. 22

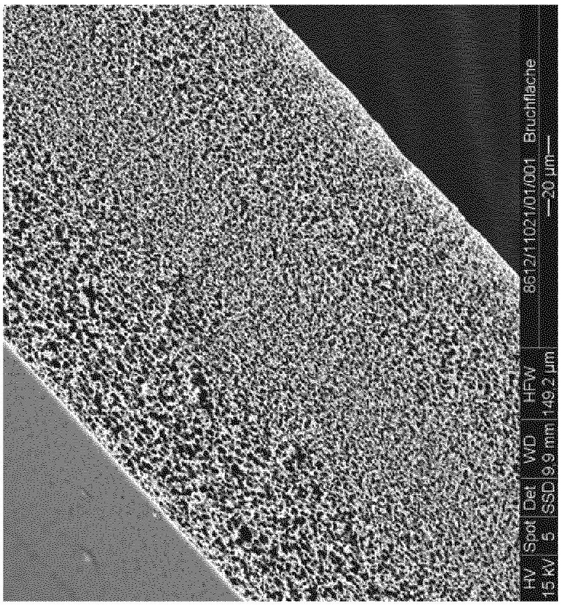


Fig. 20

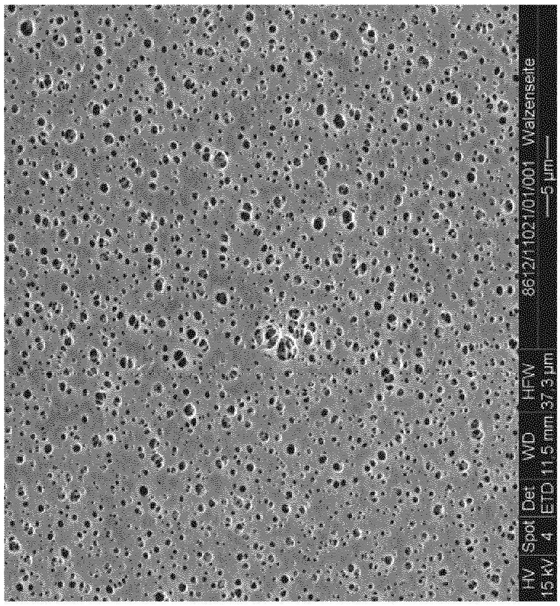


Fig. 21

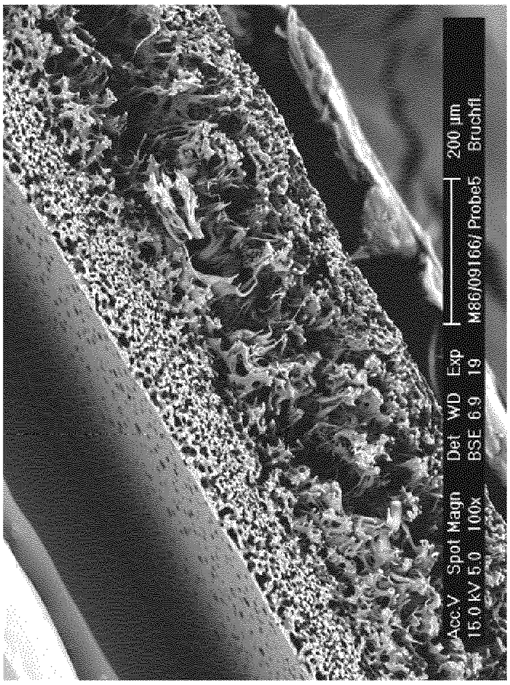


Fig. 23

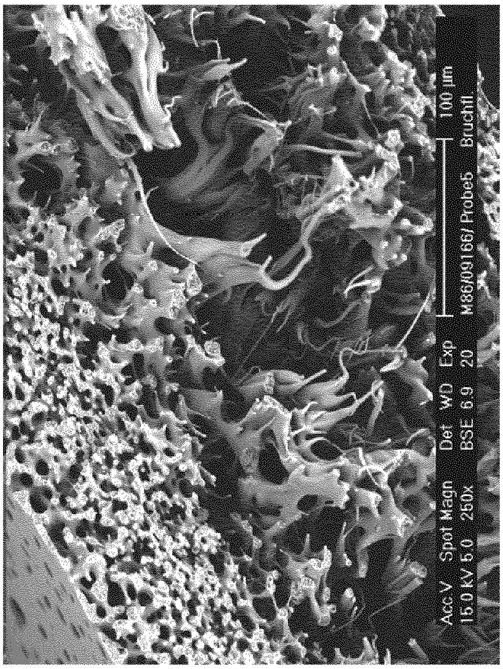


Fig. 24

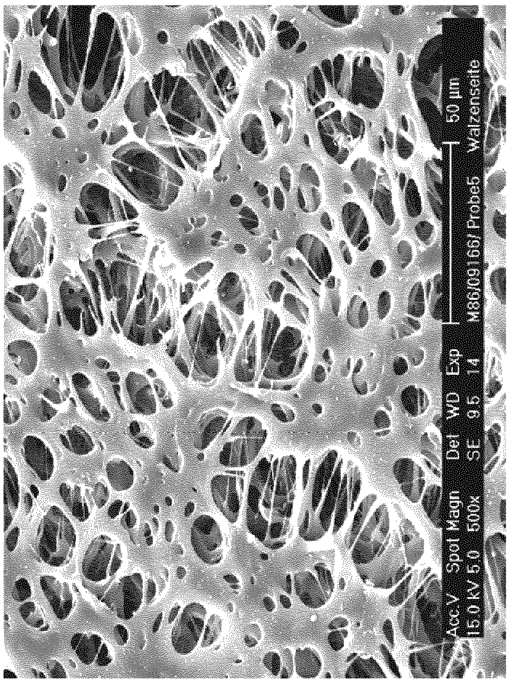


Fig. 25

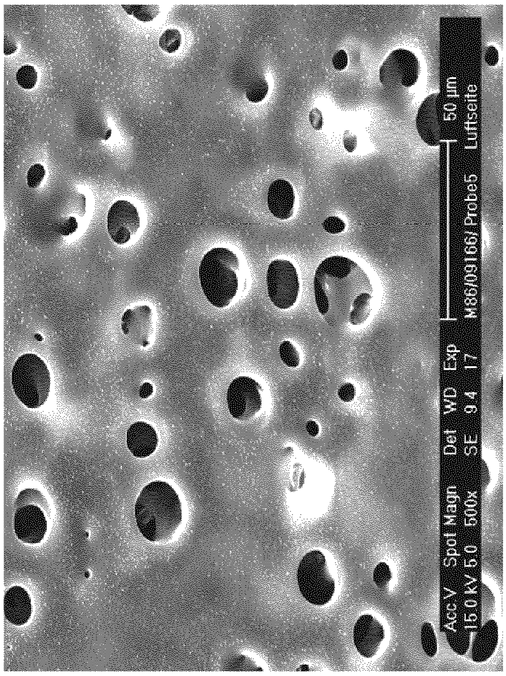


Fig. 26

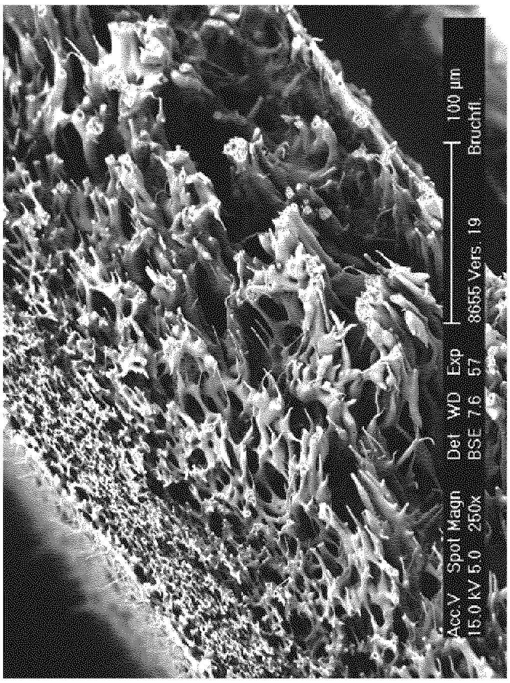


Fig. 27

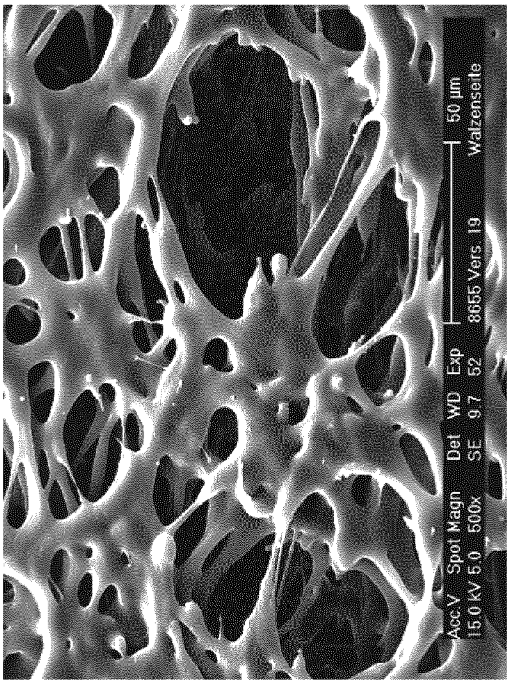


Fig. 28

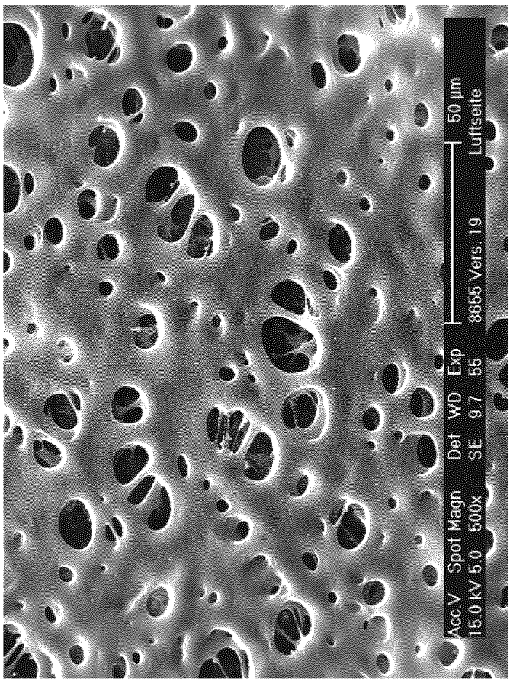


Fig. 29

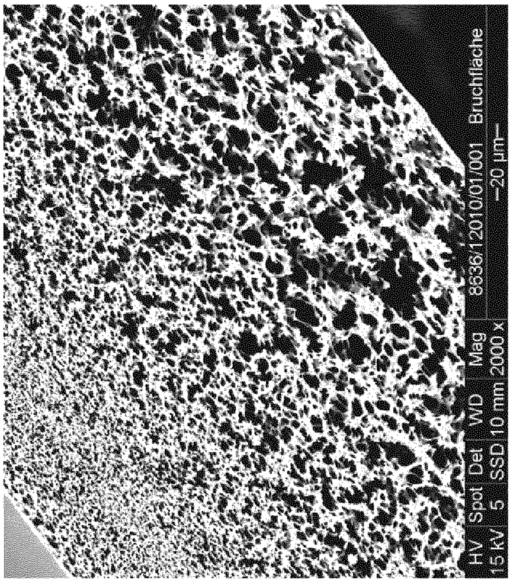


Fig. 30

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 6056903 A
- EP 0696935 A
- US 5846422 A
- US 5979670 A
- US 5906742 A
- US 5886059 A
- WO 0207854 A
- US 5240862 A
- EP 0254100 B1
- US 4943374 B1
- WO 9640421 A1
- EP 2113298 A1
- US 20100219122 A1
- JP 2008221050 A
- EP A0882494 B1
- EP 0297744 A
- EP A0543355 B1
- US 6054899 A
- EP 361085 A
- EP 0543355 A
- DE 3617724 A